# EUROPEAN PATENT APPLICATION

(11) **EP 2 502 994 A1**
(43) Date of publication of application: **26.09.2012**
(21) Application number: 10831650.6
(22) Date of filing: 19.11.2010
(51) Int. Cl.: C12N 15/09, C12Q 1/68

(54) **PRIMER SET FOR AMPLIFICATION OF MTHFR GENE, MTHFR GENE AMPLIFICATION REAGENT COMPRISING SAME, AND USE OF SAME**

(30) Priority: 19.11.2009 JP 2009264052
(71) Applicant: Arkray, Inc., Kyoto-shi, Kyoto 601-8045 (JP)
(72) Inventor: HIRAI Mitsuharu, Kiyoto-shi Kyoto 602-0008 (JP); KOZUKA Masahiro, Kyoto-shi Kyoto 602-0008 (JP)
(74) Representative: Jones, Elizabeth Louise
(86) International application number: PCT/JP2010/070669
(87) International publication number: WO 2011/062258

(57) **Abstract**

The present invention provides a primer set for specifically amplifying a target region in a MTHFR gene by a nucleic acid amplification method. A primer set including: a primer set (1) including a F1 primer and a R1 primer; and a primer set (2) including a F2 primer and a R2 primer is used. This allows two target regions respectively including sites at which two kinds of polymorphisms occur in the MTHFR gene to be amplified at the same time in the same reaction solution, for example.
(F1) a 20- to 28-mer oligonucleotide whose 3' end is guanine (g) as the 8715th base in the base sequence of SEQ ID NO: 1
(R1) an oligonucleotide complementary to an 18- to 26-mer oligonucleotide whose 5' end is cytosine(c) as the 8817th base in the base sequence of SEQ ID NO: 1
(F2) a 26- to 36-mer oligonucleotide whose 3' end is guanine (g) as the 10590th base in the base sequence of SEQ ID NO: 1
(R2) an oligonucleotide complementary to a 22- to 34-mer oligonucleotide whose 5' end is cytosine(c) as the 10695th base in the base sequence of SEQ ID NO: 1

SEQUENCE LISTING

<110>  Arkray Inc.,

<120>  Primer set for amplification of MTHFR gene, reagent for amplification
of MTHFR gene and use thereof

<130>  TF10044-00WO

<150>  JP 2009-264052
<151>  2009-11-19

<160>  56

<170>  PatentIn version 3.1

<210>  1
<211>  15835
<212>  DNA
<213>  Homo sapiens

<400>  1
aatcagaggg tttacaggtg gttcagcttc ctcctactct aggttctgtt ccagcaagca     60
attaacgagg tgcgccctta aacgctggag gaaagccaac tggctgctct tgctgttact    120
cctccccccc gcccccgttc ctcactcccc acagccatcc ccactgagaa tctggagttt    180
gaggtcagaa tgaaagagag cagccctaga gggagaaagc tttggcccag ggttcttagt    240
ctggaatcaa ctccttgtct ttggatgtat ccccgtgtag tctgtgcacc tgtgtgtgta    300
tttcagggga agggagcagt gcatttaatc agattgtcaa aagagtctaa gaccccaaat    360
ggttaggtac acagggttag tggtggacag tctgaaagaa atgaacctca cctgggcttt    420
cctctgttgt gccatgtcac cacacaccca ttcactactg tgtgtttgcc cattgctgtg    480
caagtgtttt gtttgttttt aagtgtttgt cttattttct taaccagact gccagatgac    540
cctatgccct ctgttggcct gtctgtgccc tggtggctct gattacttgt ttctgttttt    600
tgtttttgtt tttttttttg agatggagtt ttgcttttgt cgcccaggct ggagtgctat    660
ggcacaatct tggttcactg caacctctgc cttccgggtt caagcgattc tcctgcctca    720
gcctccagag tagctgggat tacaggcgcc cgccaccaaa cctggctaat ttttatattt    780
ttagtagagt cgggatttca ccatgttggc caggctggtc tcaaactcct ggcctcaggt    840
gatccaccca cctcggcctc ccaaagtgct gggattacag gtgtgttttt tgttttttta    900
agagatggag tctcgctatg ttgcccaggc tggccttgaa ctcctgggct caggcaatcc    960
ttctgcctca gccttcccag tagctgggtc actgtgatga tttgaattga attctgtgat   1020
gtgtaagaag agcagcctgc aaggcaagca cagatggggc agcttttgtt ctgagaaatt   1080
cgtgccctta ctgaacttgg gtctggctat ttttggaaca tggccagcat caagttctaa   1140
cccacaacac ggtctttttg gagtagcatg aattcaggag aaatctggct gcatagtcaa   1200
gccctcaccc cttccatcct gtgcacgaac tgtttcaagt aacagatgtt ccaggcagag   1260
ccagccagag tgagctgttc cttctctgga gggtgatctg gtatccctga acgcctgttg   1320
gcctcatctc caccaacccc tgcagtctct gcccctgagt ccccctcctt ccatccgcct   1380
ccccttacta gagcctcagc cctccctcct cgcctggaag ccttgccccc gcccccttgt   1440
gctggctgga gctcaagcct cttcctttgt cgcagctccg cccagttgaa cacacccgct   1500
ggggaaggtg cctctgttcc ctccccacgc actctgggcc tgagctgaca gagatggacc   1560
atcgaaaagc cagggtcctc ccagctgggc actactgccc ctcgctagga atatgggcct   1620
cgcaggtcgg cagcgtgagg tcctctgtgc caccttccat caggtagctg tcaccgagga   1680
gcatgttgca gtgccgggtg ggggctgcct tgcatgcaag gagcctggca gcagcggagg   1740
gcaaggcttt gagtgaggcg gcccggacag ccatagctga ggagcatgga gccactggga   1800
gggggcagtg tcaccttttt tggccttctt cctgtgtgga aatacagcgc ctccggcttg   1860
aacctgccac tcaggtgtct tgatgtgtcg ggggtgtggc tgcctgcccc ctgatgctcc   1920
ctgccccacc ctgtgcagta ggaacccagc catggtgaac gaagccagag gaaacagcag   1980
cctcaacccc tgcttggagg gcagtgccag cagtggcagt gagagctcca aagatagttc   2040
gagatgttcc accccgggcc tggaccccga gcggcatgag agactccggg agaagatgag   2100
gcggcgattg gaatctggtg acaagtggtt ctccctggaa ttcttccctc ctcgaactgc   2160
tgagggagct gtcaatctca tctcaaggta aactcatgca aggttaaggt gggaggcggg   2220
agtggtggtg cctggggagc aaactgtcag ccctgaggct gggctgccct ttaacccggc   2280
agaaaaaagc agccggaggg ggcaggaagg agaaggcaga gtgggtcatc tgatctgcaa   2340
cttctctgtt gcctgtaaaa gtccctccac agtgtcaagg tattttagac acactcttca   2400
atgggttaaa aatgttgatt ctcacactgt attctttgag ggaacttggg ggctggatca   2460
cgagtgcagt ttaggaagtc acaaagcagt gagattgttt tcagcaagtt agaaatttgg   2520
attatgcttc ttgaactcta gaaatgccaa ctgaattaca ttagcacttc atgaattgca   2580

gttttttttt taagtttatg ccttttactg tatgcacgtc tcacgtgtca ctcccaagtt 2640
tttgaagttt aaatgcacaa ggatgtttaa atacaccgtt gatagcttgt aacacaagta 2700
ctcgtgcaca tccaggcttt tttttgagtc tcgtttcgga acttggcctt ggtctgcata 2760
actgtaagct gctgcacagg gcttgggggg tggcatgaaa gaaagtgagt ctgctgaccc 2820
tctttctaac tgttcctaca gcgaggctgt gcttgccaca gttaggattc ccctaactct 2880
caatcatgat gtcttaactc acctgagata ataaacaggc aagcgctgtg aaaagtctga 2940
agtgctgctc tcacacaaaa tgatagcgct gttgttgtgt cttacctctg taatagtgat 3000
tataacggca cctcacatat actgagtatt cggtttgtac accaggcact gttctaagtt 3060
ctgtggaacc agctgccgtc atattctcct agtgttatgg ttttttgttg ttgttgttgc 3120
tttttttttt tttttttttt ttttgagaca gagtctcatt ctgtggccca ggctggagtg 3180
cagtggtgtg atcttggctc attgcaacct ctgcctcctg gctcaagcaa ttctcctgcc 3240
tcagcctctc aagtagctgg gattacaggt gcccaccacc acacccagct aatttttgta 3300
tttttagtag agatgaggtt tcactatgtt ggccaggcgg tctggaactc ctgacctcaa 3360
gtgatccact cacttcagcc tcctgaagtg ctgggattac aggcgtgagc caccacgccc 3420
agcatcctag tgttattatg aagtgggtac tactatctgc cgtgtcttat aaataaggaa 3480
acagaactca cagagattaa gtaatgtacc caagatcaca cagctataag tagcaaagcc 3540
aggatatgaa cgctgattgg tctgtctcca accttcattg atgcttctct aactcccggg 3600
ccccatggga aggcagtgac ggatggtatt tctcctggaa cctctcttca gaaacaaacc 3660
ccctacaggt ttgaccggat ggcagcaggt ggccccctct acatagacgt gacctggcac 3720
ccagcaggtg accctggctc agacaaggag acctcctcca tgatgatcgc cagcaccgcc 3780
gtgaactact gtggcctgga gaccatcctg cacatgacct gctgccgtca gcgcctggag 3840
gagatcacgg gccatctgca caaagctaag cagctgggcc tgaagaacat catggcgctg 3900
cggggaggtg tggagccagc actcccctac actctgggtt ctggctttcc cggaggccac 3960
ttggtagcct gaacttggtt cctgaaatgg tcctttactt cttcctgggc cccacgctga 4020
ggatccgcag gatcccatcc ccgccagtag tgtcagtagg cattgtgggt agtgagtcat 4080
cctgggcacc acacacaggc agccacctct tccacctctt gacctcgaca taggagttcc 4140
gtcaggaatg cagtcgcggc ctgataaccc tctgccttgc ctacaccact gcatcgattg 4200
ctggctgcac ccagcacttg gcccaatcac agagtaacag agtatccagg agtgagaata 4260
gccggtcagg cctctggtgc ccgctgttgt caggccacct aagagtggga agagggagcc 4320
tgatgggtgg gatcctatgg cctcgtgctc cctaccacaa gaccccttca cttgagatga 4380
gtagaaaatt ctcccaggag gcaggagccc caaggccgtg ggatgggaga gggtggcagg 4440
cgtgcccagg tagaagtagg gaagggctct ccaatcctcc aggcacggtg agaagcagcg 4500
gtggtctcct attctccacc gccagcacag ggtcagcatg cagtgaatat tgaactggtg 4560
ggctgctttt ttccctcctg ctgtcttacc aagtgctaga aagagacgaa ccaaacctaa 4620
ataatatgaa gtttctgcct tccagggact cgcagtcctg ggtgtcgggg ctaggtagag 4680
ggaacagaaa gggtctctgg aggttgggtg agacccagtg actatgacct ccaccaaccc 4740
tgcagaccca ataggtgacc agtgggaaga ggaggaggga ggcttcaact acgcagtgga 4800
cctggtgaag cacatccgaa gtgagtttgg tgactacttt gacatctgtg tggcaggtga 4860
gtggctggat catcctggtg gcggggatgg agctagggag gctgagatgc ccagagagcc 4920
tgtcggtgtt cggttcagga cacagtgatc aggggcactc atgggtccat gctggatgaa 4980
ggacatgcag aggtgaactg caccgtgtcc ttgctccttt tgtatcaccc actcccctcc 5040
tgggcctgcc accatcgacc cctagctttt tgttcctctg cctggctgtg cccttagcct 5100
gcttggcctt attcttttct ttaaaacgaa agaggggtct gggcgcggtg gctcacacct 5160
gtaaccccaa cactttggga ggccgaggca ggcagatcac ctgaggtcag gagttcgaga 5220
cctgcctggc caacatagcg aaaccccgtc tctactaaaa atacaaaaat tagccaggtg 5280
tggtggtgca cggctgtagt cccagctact caggaggctg agccaggaga atctcttgga 5340
cccaagaggc ggaggttgca gtgagccgag atggtgccgc tgcagtccag cctcagtggc 5400
agagcaagac tccatctcaa aaaaaaaaaa aaaacaacac cccacaaaag agggatattt 5460
actaacagga ttccagaggg cagggaaccc aagatcagga gatggcctca gacctcacaa 5520
ggcactggaa gaggtcaaaa agcaaggccc tccctcattc cctctggggc cacagggtcc 5580
cccatctccg cttcccactg cacatctgtg cttcatctgg gagacctgat acccatgctt 5640
ttccacacac ctggcctgct cctgagtgtg cagagtgtca gttccagcca gttccccagg 5700
ctaatggaca ggcctccgtt ccaaattccc aggaaagaga atcaccattg gccacagcca 5760
caagaccagg ttcccccaac agaaatgggt gggttggact tctctcccac ccttgaagct 5820
gaaggctggc attgagccct cagcgtctca gacagcgctc aggtatggca agacagtgtg 5880
caccagggac aggaagtccc gggattggca cattttctgg gatgacagtt tttttgccaa 5940
tattctggga gaaatcctac catttttata ttaaacatgg ctatgttatg gagtagcatc 6000
taaagtgata tgaactaaaa aaaaaaggcc aggtgcggtg gctgaagcct ataatcccag 6060
cactttggga ggccaaggcg ggtggatcat gagatcagga gtttgagacc atcctgtcca 6120
acatagtgaa accccgtctc tactaaaaat acaaaaatta gccgggcatg gtggcgcgtg 6180
cctgtagtcc cagctacttg gtaggctgag gcaggagaat cgcttgaacc caggaggcgg 6240
agcttgcagt gagccgagat cgcaccaatg cactgtagcc tgggcaacag agccagactc 6300
cgtctcaaaa aaaacaaaac aaaaaaccca aaaaacaaac cacgtggctt caagaaaatt 6360
acatgttgtg agtaactttg gccttgtccc taacccccga tggacatgtc cattctgtca 6420
acaggggtgt ttaggtgcta agtctgggaa gcgctgatta ggagtcagag aacagacatt 6480

tccaactgtt aaattgtcat cgcttgtatc agccattata tagtcagtta ctgggggaat 6540
atgcacccgt gatgggcgtt aggtgataat gattggaaac tagtgaagct gtgagctcac 6600
agttgctgaa tggtttctta ctttgcttat caagtgggta atgaagatga gtggattaga 6660
aaacaccaac ctgttgggca tggtagctca tgcctgtaat cccagagctt tgggagactg 6720
aggcaggaga tcaagaccag cctgggcaac acagtgagac cgtgtctcta caaaaaataa 6780
aaacatttta aaaaagaaag ccctcagctg ttcatgatag aaaaatagag taaatagcaa 6840
aatctatacg taatcaaatg ctagatttgt tcggtgatat ttaggtattg tagctatact 6900
tttcagctat taaaaagcct tgttgtttct gaaattccta ctgggaaaat agctttgtta 6960
ttgcaagtgt gaagccccat tgctttatcc cccactccct taccccttga aacaatagtt 7020
tttatattca ttttttgatg actctgggtt tcttaagaac ggaactgcct tatcagagca 7080
aagcagcttg gccttccgcc tggctcagag tcctgccctg ggcttgggct gatgaaacca 7140
gcactccatg tagtttcatg aaggcctatg gtcgtcctgc ttgcctgacc gagcaaggcg 7200
gctagtaagt ttagtcattg aggaaagggg tcagagagtt cacagagcca gtaaggagcc 7260
tatgggacat ctgatcccac ctcctaagca attctggaat cttgtcttca gcatccactg 7320
cccctgtgtt ttcagactcc aggagctcat tgttttgtga gtggcttata gtactctagc 7380
cagctctctg aaccggatgc tggttctgcc attagaaaca acacagaata tgactaataa 7440
agaacttcac tagggggctg ggtacagtgg ctcatgcctg taatctcagc actttgggag 7500
gccaaggtgg gaggattgct tgagcccaga agttccagac aaacctggac aagatagcaa 7560
gaccccatct ctaaaaaata aataattaat taaaaaaaaa ttttttaatt agccagatgt 7620
ggtggcttgc atctgtagtc caagctactc aggagactga agtgggagat cacttgtgcc 7680
cgggagttca aggctacagt gagccatgat tgtaccactg cactctagcc tgggtgacag 7740
agcgagaccc tgtctctgaa acaaaacaaa acaaactcac ctatggtgga tcctttttcc 7800
ccaggacaga cctttggaca tttgaaagga gtttctgtat tcttttcatt cttttttttt 7860
tttttttttt tgagacggag tttcactctc gttgcccagg ctagagtgca atggtacgat 7920
ctcggctcac tgcaacctct gtctcgtggg ttcaagcaat tttcctgact cagcctcctg 7980
agtagctatt acaggcatac accaccacac ctggctaatt tttgtatttt tggtagagac 8040
ggggttttac catgttggcc aggctggtct cgaactcctg acctcaggtc atcctcctgc 8100
cttggcctcc caaagtgctg ggattacaga catgagccac cgcaccctgc catccattat 8160
tttctttgaa gaaaacatca ctttcttcag ttcttccttg tgtaacttgg tttttagctt 8220
ttcactatct tggacccctg agtaatttca gtgttacatt aaaaacaatg tttaatccgg 8280
tgcctagaga aaagtcaagc ttagtacccc agatgctgcc cagccagtgc taactgtagc 8340
attttctctt ttctatggcc accaagtgca ggcctgattt gcttggctgc tcaaggcagg 8400
acagtgtggg agtttggagc aatccacccc cactcttgga actgggctct gagccacctc 8460
ccctgagagt catctctggg gtcagaagca tatcagtcat gagcccagcc actcactgtt 8520
ttagttcagg ctgtgctgtg ctgttggaag gtgcaagatc agagccccca aagcagagga 8580
ctctctctgc ccagtccctg tggtctcttc atccctcgcc ttgaacaggt ggaggccagc 8640
ctctcctgac tgtcatccct attggcaggt taccccaaag gccaccccga agcagggagc 8700
tttgaggctg acctgaagca cttgaaggag aaggtgtctg cgggagycga tttcatcatc 8760
acgcagcttt tctttgaggc tgacacattc ttccgctttg tgaaggcatg caccgacatg 8820
ggcatcactt gccccatcgt ccccgggatc tttcccatcc aggtgagggg cccaggagag 8880
cccataagct ccctccaccc cactctcacc gcaccgtcct cgcacaggct gggggctctg 8940
ggtggagtgc tgagttcgct gagttcttcc cagatctcct ctcaggtcca gaacttgcac 9000
agcgttgctt ggccacccca ttttggttac ctctaatttt ccccccaaaa cccagcaaca 9060
gtgtctgttg aggggtttgt tgtactttgg ccaacaagca tcaccaaaag ggattctaat 9120
tctcattaca aatcctgctt aaatcagtgt ttcccaaagg tggctgtcat cagaaccact 9180
tgataagctt tttcaaaaag tggatctcca ggtcccaccc ctggaggttc tcactcagta 9240
aatctgaggg aggtccttgg aatgtgtgtt ttacttcttg agcttggcag gtgattcgga 9300
tgatcagtgc agcatgggaa cagctgggtt agatgacacc atgttaccaa cagacacatc 9360
tagggctaaa aggctggctc aacccaaggc tggaatgctt agggcagctg ctctactctg 9420
taaaaagtgg gaaagctggg catagtggca cgtgcctgta gtcccggcta cttgagaagc 9480
tgaggcagga ggatggcttg agccctggcg tttgagagca gtgagactca gtctctaaaa 9540
acagaaaaaa gaggagaaaa cggcaggcca agcccaggtg tgctcctaga atatgatcgg 9600
actttcgtgg gtccctctgc ctgacactgt ggttggcatg gatgatggaa gggggagaag 9660
tatttagggt gggagacggg ctggccagca gccgccacag cccctcatgt cttggacagg 9720
gctaccactc ccttcggcag cttgtgaagc tgtccaagct ggaggtgcca caggagatca 9780
aggacgtgat tgagccaatc aaagacaacg atgctgccat ccgcaactat ggcatcgagc 9840
tggccgtgag cctgtgccag gagcttctgg ccagtggctt ggtgccaggc ctccacttct 9900
acaccctcaa ccgcgagatg gctaccacag aggtgctgaa gcgcctgggg atgtggactg 9960
aggaccccag gtgagggcag tggcccagag atccccagag gagggtccaa gagcagcccc 10020
ggctgcagtg ctggctcccc acggtggtac agatggatgt ctgtgccatg gctagctggg 10080
cacatcgaag gctccagtct cagagggcat cagatggaag aactcagcct caggggtgct 10140
cccctgcttc cggctccctc tagccaatcc cttgtctcaa ttctctgtcc ccatcctcac 10200
ccaggcgtcc cctaccctgg gctctcagcg cccaccccaa gcgccgagag gaagatgtac 10260
gtcccatctt ctgggcctcc agaccaaaga gttacatcta ccgtacccag gagtgggacg 10320
agttccctaa cggccgctgg tgagggcctg cagaccttcc ttgcaaatat atctttgttc 10380

ttgggagcgg gagggcagaa gaagtttgca tgcttgtggt tgacctggga ggagtcaggg 10440
gcagaattta caggaatggc ctcctgggca tgtggtggca ctgccctctg tcaggagtgt 10500
gccctgacct ctgggcaccc ctctgccagg ggcaattcct cttcccctgc ctttggggag 10560
ctgaaggact actacctctt ctacctgaag agcaagtccc ccaaggagga gctgctgaag 10620
atgtgggggg aggagctgac cagtgaagma agtgtctttg aagtctttgt tctttacctc 10680
tcgggagaac caaaccggaa tggtcacaaa gtgagtgatg ctggagtggg gaccctggtt 10740
catcccctgc ccctggcctg accccagctg caggccaggc tgcggggctg tgacttcccc 10800
atcctgtgcc ctcccctcca tgctgtggac atggcaaagg gagaagggta agttgggaga 10860
cctccacctg gaaggcttta gggaggcaaa gacaggctgg gtctttgttg gggccgtga 10920
gagggactca gggtgccaaa cctgatggtc gccccagcca gctcaccgtc tctcccaggt 10980
gacttgcctg ccctggaacg atgagcccct ggcggctgag accagcctgc tgaaggagga 11040
gctgctgcgg gtgaaccgcc agggcatcct caccatcaac tcacagccca acatcaacgg 11100
gaagccgtcc tccgacccca tcgtgggctg gggccccagc gggggctatg tcttccagaa 11160
ggtgtggtag ggaggcacgg ggtgcccccc tctcttgacc ggcacccgtg ggttccccag 11220
agcttagggc agagcagcct gcctctcctg ggctcttaaa ttttgaatga gcatggattg 11280
gagcggatgg gagctcatgc tttaagtcag atagacctgg ctgggcacgg aacccggcca 11340
gcactttggg aggccaaggc gggtggatca cttgagccca ggagttcgag accagcctgg 11400
gcaacatggt gaaacccatt ctctacaaaa aatacaaaaa tgaccgaggc atggtggtgt 11460
acacctgtag tcccaggtac ttagggggct ggggtgagag gatcgcttga gtccgagagg 11520
ttgaggctcc agtaagccat atttgtgcct ctgcactcca gcctggccca caaagtgaga 11580
ccctgtctca aaaaaaaaaa aaaaaaaaaa aaaaaaagat tggctgggct tggtggctca 11640
tgcctgtaat cccagcactt taggaggcca aggtgggcag atcacaaggt caggagttcg 11700
agaccagcct gacaaacatg atgaaacccc atctctacta aaaatacaac aattagccgg 11760
gtgtggtggc gggcacctgt agttgcagct acttgggagg ctgaggcagg agaatcgctt 11820
gaacccggga gacggaggtt gcagtgagct gagatcatgc cactgcactc cagcctgggc 11880
aacagagcaa gactccatct caaaaaaaaa aaaaaaaaga tagaaatcag acctatggag 11940
gcaggtcact cgacctctct gagcctggtt ttctcgtctg taaaatggga tgtagcttgt 12000
gttctgggca tgaaacctaa caaagcatgt aacgtgcttg gcacatggta agagcaggaa 12060
gcactccctg aaaggcagct gctgttacta tcctgagttg acacctttct cccagggctg 12120
ctggagttaa cattatgttc tgatctaagg cccacgggcc ctggagcaga aagacctgag 12180
tttaaggacc agctctactg tttactgtca gagtagccaa cgtgctcaac ttccctgaac 12240
ccactttcct cacgtggggc tcactgtgcc cagcctcccg tattatcagc attctagcaa 12300
atgagtttga gtaaacccag cttaatccct gataggcact ggttctagag gagatgtcaa 12360
tgtcattgtc agcttgcact ggggaggatg gaatgtgtgg ggcctctctc ccatgactcg 12420
gcctgtcaaa ccagttgctg ctgttcagaa atggcttctt cgtggagtct tccctgactg 12480
attgggagag gggtggctga tagccctcag acctgtccag actgagaaac tcacgtcccc 12540
actcccctca ccctttgctt atggacgtgc caacgccaca gttcattatt ccgcccacat 12600
ggctgccagt agtcctgata ccttaggtgt ctgcgaaagg gcgtctggca gggctggggt 12660
tggtgacagg cacctgtctc tcccacaggc ctacttagag tttttcactt cccgcgagac 12720
agcggaagca cttctgcaag tgctgaagaa gtacgagctc cgggttaatt accaccttgt 12780
caatgtgaag gtaggccagg ccccagggtt cccacagagt accaggccct tcgttgagca 12840
aacacccagc acctagcctg ggctaggcat tgtgcaagac agagagatac aggcagaact 12900
ggggccctgc tcttaaggag ctcccagcct agagtgaggg aggcagatca atgtgtgacc 12960
accagccagt gtaagtggga aaatcaagtt caggagagga tgctgtcggg tccagaggag 13020
ggatatctca cctgggaggc cagggaaggc ttctcagagg gtcatctgtg ggaatcactg 13080
aggacaaaga ggagttggcc aacaggagag aagggtattc ctgggacagg gcagaggaca 13140
ggcagaggca gtgagctgca ccatctgggg aaaccgggaa catgagcagg aggaggcggg 13200
agtgttaagc aggggccagg cccgcagaga tcatggttgt cctgctggaa tttgagtttt 13260
ccctcaatgc aagggaagcc tttctccagc agtggggtgg cttggttcct gctttatttt 13320
aggcaaatca cagtggatag tcatacggat gagagagatt ggggacggct agagccgggg 13380
aggccagtga agagacccct ggagtgaccc aagccaaaca cactcctgga ggtgggctag 13440
acaagaggga atggatcaga gagtgagcag ggggtgctca aggaggagag gggaagtggc 13500
caggacgcct ctaggttcag gcttgtgtgg ggagagatat cattcacaga tgcaggcaga 13560
ggaggccagg agcggaggca cagggagagg ggagtggagg cgcagggaga gtggaggccg 13620
ggagtggagg cgcaggcaga gggtattgag cccagacagc ctcattgagt caaggctgtc 13680
caggcagtgg gactccagtt gttcttggcc caggtcttac ccccacccca catcccctca 13740
gggtgaaaac atcaccaatg cccctgaact gcagccgaat gctgtcactt ggggcatctt 13800
ccctgggcga gagatcatcc agcccaccgt agtggatccc gtcagcttca tgttctggaa 13860
ggtaaaggag ccgggggcaa gcttgccccg cccacctgga aaaccgtggg gagggattgg 13920
gaccaagtcc caagcgtgtg ctgaaggcca cactggaccc agccttcagg gcacacccag 13980
ctctgactca cccatgtcac tgctgacgca gggtgtttat ttctgggcag gggtgggaag 14040
tgatactggc agtgggcctt gttctattcc gggaaatgtc ctgttgagca gagcccttgg 14100
agagccctgt taatcttgcc tctgtgtgtg tgtgcatgtg tgcgtgtgtg cgggggtatg 14160
tgtgtgtagg acgaggcctt tgccctgtgg attgagcggt ggggaaagct gtatgaggag 14220
gagtccccgt cccgcaccat catccagtac atccacgaca actacttcct ggtcaacctg 14280

gtggacaatg acttcccact ggacaactgc ctctggcagg tggtggaaga cacattggag 14340
cttctcaaca ggcccaccca gaatgcgaga gaaacggagg ctccatgacc ctgcgtcctg 14400
acgccctgcg ttggagccac tcctgtcccg ccttcctcct ccacagtgct gcttctcttg 14460
ggaactccac tctccttcgt gtctctccca ccccggcctc cactccccca cctgacaatg 14520
gcagctagac tggagtgagg cttccaggct cttcctggac ctgagtcggc cccacatggg 14580
aacctagtac tctctgctct agccaggagt ctgtgctctt ttggtgggga gcacttgcgt 14640
cctgcagagg accacagtgg gtggcacctc ctgagaaggc gaggagagtg gttgttgcca 14700
actaagccct cgaaccaagg cagcctccag agccagcctg ggactcccag tgaacttacc 14760
cttggagccc gtgcagtaca ggcaaaacac gcaagggcat caggcactgg tggcatcgta 14820
gaagagatgt ggcaaagtgc tgtacccttc cacctcctag aggtgggcag ctgggcccca 14880
cctacttgtg actgaagggg cacaccactg ccctgcctgc ccacttagcc gtccatggca 14940
ccagccccct ggatgggcat tgggctgaca cctaccatgc tgctttttgg cacagttgtc 15000
tattctgagc cttgagagaa aaagtgcccc ttaagggttg aaggcagtct gaacccttgt 15060
gcttggtggg gctcgtggcc ttcccctttt gcctggctgt ggaggcctga tgctgccccg 15120
ttccctgtca gaggctaaga tgagatttgc cagcacaggg gccccagatc tgcctgggcc 15180
tgtgcagcag cccagcttcc tggtgtattt ttcaggtagg cccttgtcct gccagctgcc 15240
ttcctcatcc cctcgtcctg tcccagaggt tatctgcctg gcctggctcc ccacgagtca 15300
cctgcaagcc ccagggcctg ggggcagtga ctggcaggtg cagatgggct gtttcgtgta 15360
gtggaagagc agcctgatgg ccaagggggt ggacgcaatt gtgggatgtc ctctttactc 15420
ccttcctggc ctcactggct ggggcagagg ggcagccgct aggagagact gaaagcagca 15480
gctaggactg aggagtgggt tttattgtcc ttcagagctc ttcaagctgt cccctctgtc 15540
atcactccct ggatgtgtgg ggcatggttc cttccctggg aaggctaagt tcagttctgt 15600
tttttattct atgagaacaa gtcacagctg cagctgggcc ccatgctctg ccccaagccc 15660
ccaaccccgc ggtgctccgg cggcttcctg tccactctcg gggcccttgg ggcctggctt 15720
gctccagggt cttgggctac tggcagctcc tctccttggg ctcctggctg ccaggcgttg 15780
gtgccacttc ttaaaggcct ggaaccaggg aggagaggaa atgctattgt tgtgg 15835

<210> 2
<211> 28
<212> DNA
<213> Artificial

<220>
<223> forward primer

<400> 2
cgaagcaggg agctttgagg ctgacctg 28

<210> 3
<211> 27
<212> DNA
<213> Artificial

<220>
<223> forward primer

<400> 3
gaagcaggga gctttgaggc tgacctg 27

<210> 4
<211> 26
<212> DNA
<213> Artificial

<220>
<223> forward primer

<400> 4
aagcagggag ctttgaggct gacctg 26

<210> 5

<211>  25
<212>  DNA
<213>  Artificial

<220>
<223>  forward primer

<400>  5
agcagggagc tttgaggctg acctg                                          25


<210>  6
<211>  24
<212>  DNA
<213>  Artificial

<220>
<223>  forward primer

<400>  6
gcagggagct ttgaggctga cctg                                           24


<210>  7
<211>  23
<212>  DNA
<213>  Artificial

<220>
<223>  forward primer

<400>  7
cagggagctt tgaggctgac ctg                                            23


<210>  8
<211>  22
<212>  DNA
<213>  Artificial

<220>
<223>  forward primer

<400>  8
agggagcttt gaggctgacc tg                                             22


<210>  9
<211>  21
<212>  DNA
<213>  Artificial

<220>
<223>  forward primer

<400>  9
gggagctttg aggctgacct g                                              21


<210>  10
<211>  20
<212>  DNA
<213>  Artificial

<220>

<223>  forward primer

<400>  10
ggagctttga ggctgacctg                                                    20


<210>  11
<211>  26
<212>  DNA
<213>  Artificial

<220>
<223>  reverse primer

<400>  11
ggacgatggg gcaagtgatg cccatg                                             26


<210>  12
<211>  25
<212>  DNA
<213>  Artificial

<220>
<223>  reverse primer

<400>  12
gacgatgggg caagtgatgc ccatg                                              25


<210>  13
<211>  24
<212>  DNA
<213>  Artificial

<220>
<223>  reverse primer

<400>  13
acgatggggc aagtgatgcc catg                                               24


<210>  14
<211>  23
<212>  DNA
<213>  Artificial

<220>
<223>  reverse primer

<400>  14
cgatggggca agtgatgccc atg                                                23


<210>  15
<211>  22
<212>  DNA
<213>  Artificial

<220>
<223>  reverse primer

<400>  15
gatggggcaa gtgatgccca tg                                                 22

<210>  16
<211>  21
<212>  DNA
<213>  Artificial

<220>
<223>  reverse primer

<400>  16
atggggcaag tgatgcccat g                                                21


<210>  17
<211>  20
<212>  DNA
<213>  Artificial

<220>
<223>  reverse primer

<400>  17
tggggcaagt gatgcccatg                                                  20


<210>  18
<211>  19
<212>  DNA
<213>  Artificial

<220>
<223>  reverse primer

<400>  18
ggggcaagtg atgcccatg                                                   19


<210>  19
<211>  18
<212>  DNA
<213>  Artificial

<220>
<223>  reverse primer

<400>  19
gggcaagtga tgcccatg                                                    18


<210>  20
<211>  36
<212>  DNA
<213>  Artificial

<220>
<223>  forward primer

<400>  20
ggggagctga aggactacta cctcttctac ctgaag                                36


<210>  21
<211>  35
<212>  DNA
<213>  Artificial

<220>
<223>  forward primer

<400>  21
gggagctgaa ggactactac ctcttctacc tgaag                                35


<210>  22
<211>  34
<212>  DNA
<213>  Artificial

<220>
<223>  forward primer

<400>  22
ggagctgaag gactactacc tcttctacct gaag                                 34


<210>  23
<211>  33
<212>  DNA
<213>  Artificial

<220>
<223>  forward primer

<400>  23
gagctgaagg actactacct cttctacctg aag                                  33


<210>  24
<211>  32
<212>  DNA
<213>  Artificial

<220>
<223>  forward primer

<400>  24
agctgaagga ctactacctc ttctacctga ag                                   32


<210>  25
<211>  31
<212>  DNA
<213>  Artificial

<220>
<223>  forward primer

<400>  25
gctgaaggac tactacctct tctacctgaa g                                    31


<210>  26
<211>  30
<212>  DNA
<213>  Artificial

<220>
<223>  forward primer

<400>  26

ctgaaggact actacctctt ctacctgaag 30

<210> 27
<211> 29
<212> DNA
<213> Artificial

<220>
<223> forward primer

<400> 27
tgaaggacta ctacctcttc tacctgaag 29

<210> 28
<211> 28
<212> DNA
<213> Artificial

<220>
<223> forward primer

<400> 28
gaaggactac tacctcttct acctgaag 28

<210> 29
<211> 27
<212> DNA
<213> Artificial

<220>
<223> forward primer

<400> 29
aaggactact acctcttcta cctgaag 27

<210> 30
<211> 26
<212> DNA
<213> Artificial

<220>
<223> forward primer

<400> 30
aggactacta cctcttctac ctgaag 26

<210> 31
<211> 34
<212> DNA
<213> Artificial

<220>
<223> reverse primer

<400> 31
cactccagca tcactcactt tgtgaccatt ccgg 34

<210> 32
<211> 33

<212>  DNA
<213>  Artificial

<220>
<223>  reverse primer

<400>  32
actccagcat cactcacttt gtgaccattc cgg                                    33


<210>  33
<211>  32
<212>  DNA
<213>  Artificial

<220>
<223>  reverse primer

<400>  33
ctccagcatc actcactttg tgaccattcc gg                                     32


<210>  34
<211>  31
<212>  DNA
<213>  Artificial

<220>
<223>  reverse primer

<400>  34
tccagcatca ctcactttgt gaccattccg g                                      31


<210>  35
<211>  30
<212>  DNA
<213>  Artificial

<220>
<223>  reverse primer

<400>  35
ccagcatcac tcactttgtg accattccgg                                        30


<210>  36
<211>  29
<212>  DNA
<213>  Artificial

<220>
<223>  reverse primer

<400>  36
cagcatcact cactttgtga ccattccgg                                         29


<210>  37
<211>  28
<212>  DNA
<213>  Artificial

<220>
<223>  reverse primer

<400>  37
agcatcactc actttgtgac cattccgg                                          28


<210>  38
<211>  27
<212>  DNA
<213>  Artificial

<220>
<223>  reverse primer

<400>  38
gcatcactca ctttgtgacc attccgg                                           27


<210>  39
<211>  26
<212>  DNA
<213>  Artificial

<220>
<223>  reverse primer

<400>  39
catcactcac tttgtgacca ttccgg                                            26


<210>  40
<211>  25
<212>  DNA
<213>  Artificial

<220>
<223>  reverse primer

<400>  40
atcactcact ttgtgaccat tccgg                                             25


<210>  41
<211>  24
<212>  DNA
<213>  Artificial

<220>
<223>  reverse primer

<400>  41
tcactcactt tgtgaccatt ccgg                                              24


<210>  42
<211>  23
<212>  DNA
<213>  Artificial

<220>
<223>  reverse primer

<400>  42
cactcacttt gtgaccattc cgg                                               23

<210>  43
<211>  22
<212>  DNA
<213>  Artificial

<220>
<223>  reverse primer

<400>  43
actcactttg tgaccattcc gg                                                22


<210>  44
<211>  21
<212>  DNA
<213>  Artificial

<220>
<223>  probe

<400>  44
gcgtgatgat gaaatcgrct c                                                 21


<210>  45
<211>  20
<212>  DNA
<213>  Artificial

<220>
<223>  probe

<400>  45
cgtgatgatg aaatcgrctc                                                   20


<210>  46
<211>  19
<212>  DNA
<213>  Artificial

<220>
<223>  probe

<400>  46
gtgatgatga aatcgrctc                                                    19


<210>  47
<211>  18
<212>  DNA
<213>  Artificial

<220>
<223>  probe

<400>  47
tgatgatgaa atcgrctc                                                     18


<210>  48
<211>  17
<212>  DNA
<213>  Artificial

<220>
<223>  probe

<400>  48
gatgatgaaa tcgrctc                                                    17


<210>  49
<211>  19
<212>  DNA
<213>  Artificial

<220>
<223>  probe

<400>  49
tcaaagacac ttkcttcac                                                  19


<210>  50
<211>  18
<212>  DNA
<213>  Artificial

<220>
<223>  probe

<400>  50
caaagacact tkcttcac                                                   18


<210>  51
<211>  17
<212>  DNA
<213>  Artificial

<220>
<223>  probe

<400>  51
aaagacactt kcttcac                                                    17


<210>  52
<211>  16
<212>  DNA
<213>  Artificial

<220>
<223>  probe

<400>  52
aagacacttk cttcac                                                     16


<210>  53
<211>  15
<212>  DNA
<213>  Artificial

<220>
<223>  probe

<400>  53
agacacttkc ttcac                                                      15

<210>  54
<211>  14
<212>  DNA
<213>  Artificial

<220>
<223>  probe

<400>  54
gacacttkct tcac                                                          14


<210>  55
<211>  19
<212>  DNA
<213>  Artificial

<220>
<223>  probe

<400>  55
gtgatgatga aatcgactc                                                     19


<210>  56
<211>  16
<212>  DNA
<213>  Artificial

<220>
<223>  probe

<400>  56
aagacacttg cttcac                                                        16

## Description

### Technical Field

The present invention relates to a primer set for amplifying a MTHFR gene, a MTHFR gene amplification reagent containing the primer set, and use of the primer set.

### Background Art

Homocysteine is an intermediate metabolite in the metabolic pathway of methionine. Some homocysteines are metabolized to cysteine, and some are metabolized again to methionine. It has been reported that an increase in homocysteine *in vivo* may cause cardiovascular diseases such as arteriosclerosis, coronary artery diseases, and cardiac diseases, as well as hyperhomocysteinemia. Methylenetetrahydrofolate reductase (MTHFR) is involved in the production of homocysteine. Also, the relevance of polymorphisms of the MTHFR gene with the above-described diseases also has been reported (see Non-Patent Document 1, for example).

More specifically, it is considered that homocysteine in blood is increased by the reduction in MTHFR activity caused by the polymorphisms of the MTHFR gene, resulting in the onset of the diseases. As the polymorphisms of the MTHFR gene, MTHFR*677 and MTHFR*1298 are known, for example. MTHFR*677 is a mutant MTHFR gene in which the 8747th base is thymine (t), whereas it is cytosine (c) in the wild-type MTHFR gene. MTHFR*1298 is a mutant MTHFR gene in which the 10649th base is cytosine (c), whereas it is adenine (a) in the wild-type MTHFR gene. For example, it has been reported that, when a homozygous mutation MTHFR*677 (T/T) is caused in the MTHFR gene, the MTHFR activity is reduced, whereby the amount of homocysteine is increased. On the other hand, it has been reported that, when a heterozygous mutation MTHFR*1298 (A/C) or a homozygous mutation MTHFR*1298 (C/C) is caused in the MTHFR gene, the MTHFR activity is reduced although the mechanism thereof is unknown, so that the required dose of a therapeutic agent for rheumatoid arthritis, such as methotrexate, can be reduced, for example (see Non-Patent Document 2, for example). Thus, examining a plurality of polymorphisms of the MTHFR gene is very important in order to predict the onset of diseases such as cardiovascular diseases owing to the increase in the amount of homocysteine in blood and the dose of a therapeutic agent for rheumatoid arthritis or the like.

On the other hand, polymorphism detection has been carried out widely as a method for genetic level analysis of the cause of all kinds of diseases, susceptibility to a disease (i.e., likelihood of being affected by a disease) between individuals, the difference in drug efficacy between individuals, etc. Examples of the polymorphism include a point mutation, which is a so-called single nucleotide polymorphism (SNP). In general, polymorphism detection is carried out by a direct sequencing method, RFLP (Restriction Fragment Length Polymorphism) analysis, an ASP-PCR method, or the like, for example. The direct sequencing method is carried out by, for example, amplifying a region corresponding to a detection target sequence in a target DNA in a sample by PCR (Polymerase Chain Reaction) and then analyzing the entire gene sequence of the amplification product. In the RFLP method, for example, first, a region corresponding to a detection target sequence in a target DNA in a sample is amplified by PCR. Subsequently, the thus-obtained amplification product is cleaved by a restriction enzyme that exhibits different cleaving actions depending on the presence or absence of a target mutation in the detection target sequence. Then, the typing is achieved through electrophoresis. In the ASP-PCR method, for example, PCR is carried out using a primer having a target mutation in its 3' end region, and the mutation is determined depending on whether or not the amplification occurred.

However, these methods are laborious and require high costs, because, for example, purification of DNA extracted from a sample, electrophoresis, treatment with a restriction enzyme, and the like are required. Moreover, after carrying out the PCR, the reaction vessel has to be opened once. Thus, there is a possibility that the amplification product may be mixed in a reaction system to be used subsequently, resulting in a deterioration in the accuracy of the analysis. Furthermore, since it is difficult to automate the operations, it is not possible to analyze a large number of samples by these methods. Furthermore, the ASP-PCR method also has a problem of low specificity.

In light of these problems, Tm (Melting Temperature) analysis has been put to practical use as a method for detecting a polymorphism in recent years. Tm analysis is a method for analyzing the melting temperature (Tm) of a double-stranded nucleic acid. Since the method is carried out by analyzing the melting curve of the double strand, it is also called "melting curve analysis". Tm analysis is carried out in the following manner, for example. First, using a probe complementary to a region containing a detection target polymorphism, a hybrid (double-stranded nucleic acid) of a nucleic acid to be examined (hereinafter simply referred to as a "test nucleic acid") with the probe is formed. Then, the thus-obtained hybrid is heat-treated, and dissociation (melting) of the hybrid into single-stranded nucleic acids accompanying the temperature rise is detected based on the change in a signal such as absorbance. By determining the Tm value based on the result of the detection, the polymorphism is determined. The Tm value becomes higher as the complementarity between the single-stranded nucleic acids of the hybrid becomes higher, and becomes lower as the complementarity between the same becomes lower. Thus, in the case where a polymorphism at a detection target site is X or Y, the Tm value of a hybrid composed of a nucleic acid containing the target polymorphism (e.g., Y) and a probe that is 100% complementary thereto is determined beforehand (the Tm value as an evaluation standard value). Subsequently, the Tm value of a hybrid composed of the test nucleic acid and the probe is measured (the Tm value as a measured value). Then, when this measured value is the same as the evaluation standard value, it can be determined that the test nucleic acid shows a perfect match with the probe, in other words, the detection target site in the test nucleic acid is the target polymorphism (Y). On the other hand, when the measured value is lower than the evaluation standard value, it can be determined that the test nucleic acid shows a mismatch with the probe, in other words, the detection target site in the test nucleic acid is the other polymorphism (X). According to such a method, a polymorphism can be detected merely by heat-treating a PCR reaction solution containing the probe and then measuring signals, for example. Thus, automation of a detection device is also possible.

However, the detection method utilizing such Tm analysis also has a problem in that, for example, in PCR, a region including the target polymorphism has to be amplified specifically and efficiently. In particular, when a plurality of gene polymorphisms are present, a large amount of labour is required to analyze even a single sample. Thus, there also is a problem in that it is not practical to analyze a large number of samples.

### Citation List

### Non-Patent Document(s)

[Non-Patent Document 1] Fukuzawa et al., "Tokusyu Kouketsuatsu Saishin no Kenkyu Douko Kiso-hen (The Latest Trend in Hypertension Research, Basic Edition)", Japanese Journal of Clinical Medicine, Nippon Rinsho, July 2006, vol. 64, suppl. 5, pp. 173 to 176
[Non-Patent Document 2] Urano et al., "Polymorphisms in the methylenetetrahydrofolate reductase gene were associated with both the efficacy and the toxicity of methotrexate used for the treatment of rheumatoid arthritis, as evidenced by single locus and haplotype analyses. ", Pharmacogenetics, April 2002, 12(3), pp. 183 to 190

### Brief Summary of the Invention

### Problem to be Solved by the Invention

With the foregoing in mind, it is an object of the present invention to provide a primer and a primer set for specifically amplifying a target region in a MTHFR gene by a nucleic acid amplification method.

In order to achieve the above object, the present invention provides a primer for amplifying a MTHFR gene. The primer is composed of an oligonucleotide (F1), an oligonucleotide (R1), an oligonucleotide (F2), or an oligonucleotide (R2) shown below:
(F1) a 20- to 28-mer oligonucleotide whose 3' end is guanine (g) as the 8715th base in the base sequence of SEQ ID NO: 1;
(R1) an oligonucleotide complementary to an 18- to 26-mer oligonucleotide whose 5' end is cytosine(c) as the 8817th base in the base sequence of SEQ ID NO: 1;
(F2) a 26- to 36-mer oligonucleotide whose 3' end is guanine (g) as the 10590th base in the base sequence of SEQ ID NO: 1; and
(R2) an oligonucleotide complementary to a 22- to 34-mer oligonucleotide whose 5' end is cytosine(c) as the 10695th base in the base sequence of SEQ ID NO: 1.

The present invention also provides a primer set for amplifying a MTHFR gene. The primer set includes at least one of primer set (1) and primer set (2). Primer set (1) includes at least one of a primer composed of oligonucleotide (F1) and a primer composed of oligonucleotide (R1). Primer set (2) includes at least one of a primer composed of oligonucleotide (F2) and a primer composed of oligonucleotide (R2).

The present invention also provides a gene amplification reagent containing the MTHFR gene amplification primer or primer set according to the present invention.

The present invention also provides a method for amplifying a MTHFR gene, including the step of: in a reaction system, amplifying a MTHFR gene using the MTHFR gene amplification primer or primer set according to the present invention with a nucleic acid in a sample as the template.

The present invention also provides a method for detecting an amplification product of a MTHFR gene, including the step of:
(A) amplifying a MTHFR gene by the amplification method according to the present invention.

The present invention also provides a method for detecting a polymorphism in a MTHFR gene, including the step of:
(A) amplifying a MTHFR gene by the amplification method according to the present invention.

### Effects of the Invention

According to the primer and primer set of the present invention, a target region including a detection target site (e.g., MTHFR*677 or MTHFR*1298) in a MTHFR gene can be amplified specifically.

### Brief Description of Drawings

[FIG. 1] FIG. 1 shows graphs showing the results of Tm analysis obtained in Example 1 of the present invention.
[FIG. 2] FIG. 2 shows graphs showing the results of Tm analysis obtained in Example 2 of the present invention.

### Mode for Carrying out the Invention

In the present invention, a detection target polymorphism in a MTHFR gene is, for example, a polymorphism of at least one of the 8747th base and the 10649th base in the base sequence of the MTHFR gene shown in SEQ ID NO: 1. The 8747th base (y) in the base sequence of SEQ ID NO: 1 is cytosine (c) when the MTHFR gene is wild-type, and thymine (t) when the MTHFR gene is mutant type. Hereinafter, this polymorphism is referred to as MTHFR*677. A homozygote with wild-type alleles is referred to as MTHFR*677 (C/C) or 8747 (C/C), a homozygote with mutant alleles is referred to as MTHFR*677 (T/T) or 8747 (T/T), and a heterozygote is referred to as MTHFR*677 (C/T) or 8747 (C/T). Furthermore, the 10649th base (m) in the base sequence of SEQ ID NO: 1 is adenine (a) when the MTHFR gene is wild-type, and cytosine (c) when the MTHFR gene is mutant type. Hereinafter, this polymorphism is referred to as MTHFR*1298. A homozygote with wild-type alleles is referred to as MTHFR*1298 (A/A) or 10649 (A/A), a homozygote with mutant alleles is referred to as MTHFR*1298 (C/C) or 10649 (C/C), and a heterozygote is referred to as MTHFR*1298 (A/C) or 10649 (A/C). The term "wild-type" also can be referred to as "normal".

The base sequence of the MTHFR gene is registered under NCBI Accession No. AY338232, for example. The base sequence of SEQ ID NO: 1 is a full-length sequence of the MTHFR gene in the base sequence of NCBI Accession No. AY338232.

In the present invention, sites at which the above-described polymorphisms occur, e.g., the 8747th and 10649th bases in the sequence of SEQ ID NO: 1 (sense strand), or bases to be paired with the 8747th and 10649th bases in the sense strand in a sequence complementary thereto (antisense strand), are referred to as "detection target sites". Furthermore, in the present invention, a region to be amplified with the primer or primer set according to the present invention is referred to as "a target region or an amplification region". The target region includes the detection target site. The target region may be, for example, a region in the sense strand of the MTHFR gene, a region in the antisense strand of the MTHFR gene, or both of them. In the present invention, the terms "sense strand" and "antisense strand" encompass, for example, amplification products of the sense strand and amplification products of the antisense strand, respectively.

In the present invention, a region in the MTHFR gene including the detection target site and to which a polymorphism detection probe to be described below can hybridize is referred to as a "detection target sequence or hybridization region". Regarding the detection target sequence, the one showing a perfect match with the probe is referred to as a "perfect match sequence", and the one showing a mismatch with the probe is referred to as a "mismatch sequence". In the present invention, the term "perfect match" means that the base at the detection target site is complementary to a base to be paired therewith in the probe, and preferably means that the detection target sequence is perfectly complementary to the probe. In the present invention, the term "mismatch" means that the base at the detection target site is not complementary to a base to be paired therewith in the probe, and preferably means that the detection target sequence is perfectly complementary to the probe except for the detection target site.

In the present invention, the ends of a base sequence means the endmost bases on the 5' side and the 3' side in the base sequence. A 5' end region is a region including several bases from the 5' end in a base sequence, and a 3' end region is a region including several bases from the 3' end in a base sequence. The several bases mean, for example, one to ten bases, one to four bases, one to three bases, and one to two bases, including the base at the end. In the present invention, the Zth base (Z is a positive integer) from an end of a base sequence is a numerical order counted with the base at the end as the first base. For example, the first base from the end means the base at the end, and the second base from the end means the base next to the base at the end.

### <MTHFR Gene Amplification Primer and Primer Set>

As described above, the primer according to the present invention includes a primer for amplifying a MTHFR gene, which is composed of an oligonucleotide (F1), an oligonucleotide (R1), an oligonucleotide (F2), or an oligonucleotide (R2) shown below:
(F1) a 20- to 28-mer oligonucleotide whose 3' end is guanine (g) as the 8715th base in the base sequence of SEQ ID NO: 1;
(R1) an oligonucleotide complementary to an 18- to 26-mer oligonucleotide whose 5' end is cytosine(c) as the 8817th base in the base sequence of SEQ ID NO: 1;
(F2) a 26- to 36-mer oligonucleotide whose 3' end is guanine (g) as the 10590th base in the base sequence of SEQ ID NO: 1; and
(R2) an oligonucleotide complementary to a 22- to 34-mer oligonucleotide whose 5' end is cytosine(c) as the 10695th base in the base sequence of SEQ ID NO: 1.

As described above, the primer set for amplifying a MTHFR gene according to the present invention includes at least one of primer set (1) and primer set (2).

According to the primer of the present invention or a primer set including the same, for example, it is possible to amplify a target region in a MTHFR gene specifically. The MTHFR gene amplification primer and primer set of the present invention also can be referred to as a MTHFR gene amplification primer reagent, for example.

As described above, according to the primer and primer set of the present invention, a target region including MTHFR*677 or MTHFR*1298 as the detection target site in the MTHFR gene can be amplified specifically and also with high efficiency, for example. Since the target region in the MTHFR gene can be amplified specifically as described above, it is also possible to detect a polymorphism with high accuracy, for example. Specifically, for example, by also carrying out Tm analysis with respect to the obtained amplification product using a probe that can hybridize to the detection target site, it becomes possible to detect the polymorphism of the detection target site with higher accuracy and to determine the genotype of polymorphisms. Moreover, for example, since the amplification of the target region and the polymorphism detection can be carried out in a single reaction system, it becomes possible to automate the operations. Furthermore, by using the primer and primer set of the present invention, for example, even when the sample is a sample containing contaminants, such as whole blood and oral mucosa, a pretreatment for, e.g., removing the contaminants can be omitted, so that the amplification reaction can be carried out more rapidly and more easily. Still further, by using the primer and primer set of the present invention, the amplification reaction can be carried out with higher amplification efficiency than in conventional art, so that it becomes possible to shorten the amplification reaction. Therefore, according to the primer and primer set of the present invention; a reagent containing the same; and an amplification method, an amplification product detection method, and a polymorphism detection method using the primer, primer set, or reagent, it is possible to analyze a polymorphism in a MTHFR gene rapidly and easily. Thus, it can be said that the present invention may be very effective in the medical field.

The MTHFR gene amplification primer set according to the present invention may include either one of primer sets (1) and (2), or may include both primer sets (1) and (2), for example. As will be described below, a target region that can be amplified specifically by primer set (1) is a region including a site at which the polymorphism MTHFR*677 occurs in the MTHFR gene, and a target region that can be amplified specifically by primer set (2) is a region including a site at which the polymorphism MTHFR*1298 occurs in the MTHFR gene. When the MTHFR gene amplification primer set according to the present invention includes both primer sets (1) and (2), for example, a target region including the site at which the polymorphism MTHFR*677 occurs and a target region including the site at which the polymorphism MTHFR*1298 occurs in the MTHFR gene can be amplified at the same time in the same reaction system.

As described above, it is known that these two kinds of polymorphisms in the MTHFR gene influence the amount of homocysteine in vivo. It is thus considered important to check both kinds of polymorphisms, rather than checking only one of them. However, according to conventional methods, it is difficult to detect a plurality of sequences in a single reaction system. Thus, in order to check both kinds of polymorphisms. i.e., MTHFR*677 and MTHFR*1298, in the MTHFR gene, it is necessary to amplify regions respectively including the sites at which the polymorphisms occur separately in different reaction systems, and analyze the obtained amplification products separately. As described above, according to conventional methods, it is very difficult to use only the MTHFR gene as a template and to amplify two kinds of target regions respectively including the sites at which the polymorphisms occur in the MTHFR gene specifically. Moreover, since considerable labour is required to analyze even a single sample as described above, there also is a problem in that it is not practical to analyze a large number of samples. In contrast, according to the primer set of the present invention, even when the primer set includes both primer sets (1) and (2), the respective target regions can be amplified at the same time and specifically in the same reaction system. Thus, in contrast to the above-described conventional methods, it is possible to reduce the labour and cost. Furthermore, since two target regions are amplified specifically in the same reaction system as described above, for example, by carrying out Tm analysis using probes that can hybridize to the detection target sites in the respective target regions, it becomes possible to detect the two kinds of polymorphisms respectively and also to determine the genotype. As described above, since the two kinds of polymorphisms in the MTHFR gene can be analyzed in the same reaction system, it is preferable not only that the primer set of the present invention includes either one of primer sets (1) and (2) but also that the primer set of the present invention includes both primer sets (1) and (2), for example. Therefore, not only by amplifying either one of the target regions but also by amplifying both the two target regions at the same time using the primer set of the present invention, the polymorphisms of the MTHFR gene can be detected more efficiently than conventional methods.

Hereinafter, a forward primer may also be referred to as a "F primer", and a reverse primer may also be referred to as a "R primer".

As described above, primer set (1) includes at least one of a primer composed of the following oligonucleotide (F1) and a primer composed of the following oligonucleotide (R1):
(F1) a 20- to 28-mer oligonucleotide whose 3' end is guanine (g) as the 8715th base in the base sequence of SEQ ID NO: 1; and
(R1) an oligonucleotide complementary to an 18- to 26-mer oligonucleotide whose 5' end is cytosine(c) as the 8817th base in the base sequence of SEQ ID NO: 1.

The primer composed of oligonucleotide (F1) is a forward primer, and also is referred to as a "F1 primer" hereinafter. The sequence of the F1 primer is the same as a partial sequence in the base sequence of SEQ ID NO: 1. That is, the sequence of the F1 primer is the same as a partial sequence in the sense strand of the MTHFR gene, so that the F1 primer can anneal to the antisense strand.

The primer composed of oligonucleotide (R1) is a reverse primer, and also is referred to as a "R1 primer" hereinafter. The R1 primer is complementary to a partial sequence in the base sequence of SEQ ID NO: 1. That is, the sequence of the R1 primer is the same as a partial sequence in the antisense strand of the MTHFR gene, so that the R1 primer can anneal to the sense strand.

Primer set (1) may include only either the F1 primer or the R1 primer, and preferably includes both the F1 primer and the R1 primer, for example.

Primer set (1) is a primer set for amplifying: a nucleic acid sequence including a region from the 8716th to 8816th bases in SEQ ID NO: 1; and a strand complementary thereto. The 8747th base in this region, i.e., the 8747th base in SEQ ID NO: 1, is a site at which the above-described polymorphism MTHFR*677 occurs. Primer set (1) also is referred to as the "MTHFR*677 primer set" hereinafter. When only the polymorphism MTHFR*677 is to be analyzed, only the MTHFR*677 primer set may be used.

In primer set (1), the F1 primer and the R1 primer each can be any primer, as long as the base at the 3' end that serves to determine the site from which DNA polymerase starts amplification satisfies the above-described condition. By fixing the base at the 3' end of each primer as described above, it is possible to sufficiently prevent primer set (1) from binding to another similar sequence, for example.

As described above, since the F1 primer and R1 primer each can be any primer as long as the base at the 3' end is fixed, the length itself of each primer is not particularly limited and can be adjusted as appropriate to a common length. The length of each primer is in the range from, for example, 13- to 50-mer, preferably 14- to 45-mer, and more preferably 15 to 40-mer. A specific example is such that: the F1 primer is an oligonucleotide whose 3' end is guanine (g) as the 8715th base in the base sequence of SEQ ID NO: 1, and the base length thereof is, for example, 20- to 28-mer, preferably 21- to 26-mer, and more preferably 22- to 24-mer; and the R1 primer is an oligonucleotide complementary to an oligonucleotide whose 5' end is cytosine (c) as the 8817th base in the base sequence of SEQ ID NO: 1, and the base length thereof is, for example, 18- to 26-mer, preferably 19- to 25-mer, and more preferably 21- to 23-mer. Since the 3' end of each of the F1 primer and the R1 primer is fixed, a region to be elongated from the primer is, for example, a region from the 8716th to 8816th bases in SEQ ID NO: 1 as described above, and the length of the obtained amplification product as a whole varies in accordance with the length of the primer.

The sequence of the F1 primer may or may not have perfect identity with a partial sequence in the base sequence of SEQ ID NO: 1, for example. In other words, the sequence of the F1 primer may or may not have perfect identity with a partial sequence of the sense strand of the MTHFR gene. As a specific example of the latter, when the F1 primer is compared with a partial sequence of the sense strand, one to five bases located at any site excluding the 3' end of the F1 primer may be different from the corresponding bases in the partial sequence, for example.

The R1 primer may or may not be perfectly complementary to a partial sequence in the base sequence of SEQ ID NO: 1. In other words, the sequence of the R1 primer may or may not have perfect identity with a partial sequence of the antisense strand of the MTHFR gene. As a specific example of the latter, when the R1 primer is compared with a partial sequence of the antisense strand, one to five bases located at any site excluding the 3' end of the R1 primer may be different from the corresponding bases in the partial sequence, for example.

Specific examples of the F1 primer and R1 primer are shown below. It is to be noted, however, that the present invention is not limited thereto.

**[Table 1]**

| Primer | Sequence | Tm(°C) | SEQ ID NO |
|---|---|---|---|
| | 5'-cgaagcagggagctttgaggctgacctg-3' | 66 | 2 |
| | 5'-gaagcagggagctttgaggctgacctg-3' | 64.4 | 3 |
| | 5'-aagcagggagctttgaggctgacctg-3' | 64.1 | 4 |
| | 5'-agcagggagctttgaggctgacctg-3' | 64 | 5 |
| F1 primer for MTHFR*677 | 5'-gcagggagctttgaggctgacctg-3' | 63.1 | 6 |
| | 5'-cagggagctttgaggctgacctg-3' | 60.9 | 7 |
| | 5'-agggagctttgaggctgacctg-3' | 60 | 8 |
| | 5'-gggagctttgaggctgacctg-3' | 58.9 | 9 |
| | 5'-ggagctttgaggctgacctg-3' | 56.8 | 10 |
| | 5'-ggacgatggggcaagtgatgcccatg-3' | 65.1 | 11 |
| | 5'-gacgatggggcaagtgatgcccatg-3' | 63.6 | 12 |
| | 5'-acgatggggcaagtgatgcccatg-3' | 63.2 | 13 |
| | 5'-cgatggggcaagtgatgcccatg-3' | 62.1 | 14 |
| R1 primer for MTHFR*677 | 5'-gatggggcaagtgatgcccatg-3' | 60 | 15 |
| | 5'-atggggcaagtgatgcccatg-3' | 59.3 | 16 |
| | 5'-tggggcaagtgatgcccatg-3' | 59.1 | 17 |
| | 5'-ggggcaagtgatgcccatg-3' | 57.6 | 18 |
| | 5'-gggcaagtgatgcccatg-3' | 55.2 | 19 |

The combination of the F1 primer and the R1 primer is by no means limited. Specifically, for example, a primer set including a F1-1 primer composed of an oligonucleotide of SEQ ID NO: 7 and a R1-1 primer composed of an oligonucleotide of SEQ ID NO: 15 is particularly preferable. In the above table, "Tm (°C)" indicates a Tm value (°C) of a hybrid of each sequence shown in the table with a sequence perfectly complementary thereto, and is calculated using MELTCALC software (http://www.meltcalc.com/) with predetermined parameters (the same applies hereinafter). The parameters were: an oligonucleotide concentration of 0.2 µmol/l; and a sodium equivalent (Na eq.) of 50 mmol/l. The Tm value can be calculated using conventionally known MELTCALC software (http://www.meltcalc.com/) or the like, for example. Also, the Tm value can be determined by a nearest neighbour method (the same applies hereinafter).

Next, as described above, primer set (2) includes at least one of a primer composed of the following oligonucleotide (F2) and a primer composed of the following oligonucleotide (R2):
(F2) a 26- to 36-mer oligonucleotide whose 3' end is guanine (g) as the 10590th base in the base sequence of SEQ ID NO: 1; and
(R2) an oligonucleotide complementary to a 22- to 34-mer oligonucleotide whose 5' end is cytosine(c) as the 10695th base in the base sequence of SEQ ID NO: 1.

The primer composed of oligonucleotide (F2) is a forward primer, and also is referred to as a "F2 primer" hereinafter. The sequence of the F2 primer is the same as a partial sequence in the base sequence of SEQ ID NO: 1. That is, the sequence of the F2 primer is the same as a partial sequence in the sense strand of the MTHFR gene, so that the F2 primer can anneal to the antisense strand.

The primer composed of oligonucleotide (R2) is a reverse primer, and also is referred to as a "R2 primer" hereinafter. The R2 primer is complementary to a partial sequence in the base sequence of SEQ ID NO: 1. That is, the sequence of the R2 primer is the same as a partial sequence in the antisense strand of the MTHFR gene, so that the R2 primer can anneal to the sense strand.

Primer set (2) may include only either the F2 primer or the R2 primer, and preferably includes both the F2 primer and the R2 primer, for example.

Primer set (2) is a primer set for amplifying: a nucleic acid sequence including a region from the 10591st to 10694th bases in SEQ ID NO: 1; and a strand complementary thereto. The 10649th base in this region, i.e., the 10649th base in SEQ ID NO: 1, is a site at which the above-described polymorphism MTHFR*1298 occurs. Primer set (2) also is referred to as the "MTHFR*1298 primer set" hereinafter. When only the polymorphism MTHFR*1298 is to be analyzed, only the MTHFR*1298 primer set may be used.

For the same reason as stated above regarding primer set (1), the F2 primer and R2 primer in primer set (2) each can be any primer, as long as the base at the 3' end satisfies the above-described condition. Thus, the length itself of each of the F2 primer and R2 primer is not particularly limited, and can be adjusted as appropriate to a length as described above. The length of each primer is in the range from, for example, 13- to 50-mer, preferably 14-to 45-mer, and more preferably 15 to 40-mer. A specific example is such that: the F2 primer is an oligonucleotide whose 3' end is guanine (g) as the 10590th base in the base sequence of SEQ ID NO: 1, and the base length thereof is, for example, 26- to 36-mer, preferably 28- to 34-mer, and more preferably 30- to 32-mer; and the R2 primer is an oligonucleotide complementary to an oligonucleotide whose 5' end is cytosine (c) as the 10695th base in the base sequence of SEQ ID NO: 1, and the base length thereof is, for example, 22- to 34-mer, preferably 26- to 32-mer, and more preferably 27- to 29-mer. Since the 3' end of each of the F2 primer and the R2 primer is fixed, a region to be elongated from the primer is, for example, a region from the 10591st to the 10694th bases in SEQ ID NO: 1 as described above, and the length of the obtained amplification product as a whole varies in accordance with the length of the primer.

The sequence of the F2 primer may or may not have perfect identity with a partial sequence in the base sequence of SEQ ID NO: 1. In other words, the sequence of the F2 primer may or may not have perfect identity with a partial sequence of the sense strand of the MTHFR gene. As a specific example of the latter, when the F2 primer is compared with a partial sequence of the sense strand, one to five bases located at any site excluding the 3' end of the F2 primer may be different from the corresponding bases in the partial sequence, for example.

The R2 primer may or may not be perfectly complementary to a partial sequence in the base sequence of SEQ ID NO: 1. In other words, the sequence of the R2 primer may or may not have perfect identity with a partial sequence of the antisense strand of the MTHFR gene. As a specific example of the latter, when the R2 primer is compared with a partial sequence of the antisense strand, one to five bases located at any site excluding the 3' end of the R2 primer may be different from the corresponding bases in the partial sequence, for example.

Specific examples of the F2 primer and the R2 primer are shown below. It is to be noted, however, that the present invention is not limited thereto.

**[Table 2]**

| Primer | Sequence | Tm(°C) | SEQ ID NO |
|---|---|---|---|
| | 5'-ggggagctgaaggactactacctcttctacctgaag-3' | 65 | 20 |
| | 5'-gggagctgaaggactactacctcttctacctgaag-3' | 63 9 | 21 |
| | 5'-ggagctgaaggactactacctcttctacctgaag-3' | 62 7 | 22 |
| | 5'-gagctgaaggactactacctcttctacctgaag-3' | 61 5 | 23 |
| | 5'-agctgaaggactactacctcttctacctgaag-3' | 61.1 | 24 |
| F2 primer for MTHFR*1298 | 5'-gctgaaggactactacctcttctacctgaag-3' | 60.4 | 25 |
| | 5'-ctgaaggactactacctcttctacctgaag-3' | 58.5 | 26 |
| | 5'-tgaaggactactacctcttctacctgaag-3' | 57 9 | 27 |
| | 5'-gaaggactactacctcttctacctgaag-3' | 56 9 | 28 |
| | 5'-aaggactactacctcttctacctgaag-3' | 56 2 | 29 |
| | 5'-aggactactacctcttctacctgaag-3' | 55.8 | 30 |
| | 5'-cactccagcatcactcactttgtgaccattccgg-3' | 66.4 | 31 |
| | 5'-actccagcatcactcactttgtgaccattccgg-3' | 66 | 32 |
| | 5'-ctccagcatcactcactttgtgaccattccgg-3' | 65.3 | 33 |
| | 5'-tccagcatcactcactttgtgaccattccgg-3' | 65 | 34 |
| | 5'-ccagcatcactcactttgtgaccattccgg-3' | 64 4 | 35 |
| | 5'-cagcatcactcactttgtgaccattccgg-3' | 63.1 | 36 |
| R2 primer for MTHFR*1298 | 5'-agcatcactcactttgtgaccattccgg-3' | 62.5 | 37 |
| | 5'-gcatcactcactttgtgaccattccgg-3' | 61.7 | 38 |
| | 5'-catcactcactttgtgaccattccgg-3' | 59 7 | 39 |
| | 5'-atcactcactttgtgaccattccgg-3' | 58.8 | 40 |
| | 5'-tcactcactttgtgaccattccgg-3' | 58.7 | 41 |
| | 5'-cactcactttgtgaccattccgg-3' | 57 7 | 42 |
| | 5'-actcactttgtgaccattccgg-3' | 56 7 | 43 |

The combination of the F2 primer and the R2 primer is by no means limited. Specifically, for example, a primer set including a F2-1 primer composed of an oligonucleotide of SEQ ID NO: 25 and a R2-1 primer composed of an oligonucleotide of SEQ ID NO: 38 is particularly preferable.

Furthermore, each of the primers included in the above-described primer sets (1) and (2) may include any conventionally known sequence added to its 5' end in order to raise the reaction temperature of an amplification reaction, for example.

The MTHFR gene amplification primer set according to the present invention preferably is used at the time of amplifying a MTHFR gene in a biological sample, for example. The biological sample is not particularly limited, and examples thereof include a whole blood sample. In particular, when the MTHFR gene amplification primer set according to the present invention is used together with a polymorphism detection probe as will be described below, the amount of the whole blood sample (the content of the whole blood) in a reaction system of an amplification reaction preferably is set to 0.1 to 0.5 vol%, for example. This will be described below.

### <Amplification Method>

As described above, the amplification method according to the present invention is a method for amplifying a MTHFR gene, including the step of: in a reaction system, amplifying a MTHFR gene using the MTHFR gene amplification primer or primer set according to the present invention with a nucleic acid in a sample as the template.

By carrying out an amplification reaction using the MTHFR gene amplification primer or primer set according to the present invention, a target region in the MTHFR gene can be amplified, as described above. When the MTHFR gene amplification primer set according to the present invention includes both primer sets (1) and (2), for example, a target region including a site at which the polymorphism MTHFR*677 occurs and a target region including a site at which the polymorphism MTHFR*1298 occurs in the MTHFR gene can be amplified at the same time in the same reaction system. The amplification method of the present invention may be characterized in that the primer or primer set of the present invention is used, and the kind, conditions, etc. of the nucleic acid amplification method are by no means limited.

In the amplification step, the MTHFR gene can be amplified by an amplification method using, as a primer, the primer or primer set of the present invention. The nucleic acid amplification method is not particularly limited as described above, and examples thereof include a PCR (Polymerase Chain Reaction) method, a NASBA (Nucleic Acid Sequence Based Amplification) method, a TMA (Transcription-Mediated Amplification) method, and a SDA (Strand Displacement Amplification) method. Among them, the PCR method is preferable.

The reaction system of the amplification reaction in the amplification step may be a reaction solution, for example. The reaction system contains, for example, the primer or primer set of the present invention and a sample, and may further contain a solvent, various components used for nucleic acid amplification, and the like.

A sample to which the present invention is applicable is not particularly limited, and examples thereof include samples containing a nucleic acid that serves as a template. The present invention preferably is applied to a sample containing contaminants, for example. Examples of the sample containing contaminants include biological samples. Examples of the biological sample include: whole blood; oral cells (e.g., oral mucosa); somatic cells such as cells of a nail and cells of a hair; germ cells; sputum; amniotic fluid; paraffin-embedded tissues; urine; gastric juice (e.g., liquid obtained by gastrolavage); and suspensions thereof. According to the amplification method of the present invention, for example, even when a sample containing various contaminants, in particular, a biological sample such as whole blood or oral cells is used, the method is less susceptible to the influence of the contaminants. Thus, according to the present invention, the target region in the MTHFR gene can be amplified specifically. According to the present invention, even a sample with a large amount of contaminants can be used as it is without being pretreated so as to remove the contaminants or so as to be purified, for example, whereas amplification using such a sample has been difficult according to conventional methods. Therefore, for example, also from the viewpoint of the pretreatment and the like of the sample, it can be said that the present invention can amplify a MTHFR gene still more rapidly than any conventional method.

The amount of the sample in the reaction system is not particularly limited. When the sample is a biological sample, for example, the lower limit of the amount of the sample in the reaction system is, for example, 0.01 vol% or more, preferably 0.05 vol% or more, and more preferably 0.1 vol% or more, and the upper limit of the same is, for example, 2 vol% or less, preferably 1 vol% or less, and more preferably 0.5 vol% or less. Specifically, when the biological sample is a whole blood sample, the amount of the whole blood sample in the reaction system is the same as described above, for example.

When the reaction system after the amplification reaction is subjected to, for example, optical detection to be described below, the amount of the biological sample in the reaction system preferably is set to 0.1 to 0.5 vol%, for example. The biological sample is not particularly limited, and may be a whole blood sample, for example. The amount of a whole blood sample in the reaction system can also be represented by percent by weight of hemoglobin (hereinafter referred to as "Hb"), instead of percent by volume (e.g., 0.1 to 0.5 vol%) as in the above. In this case, the amount of the whole blood sample in the reaction system preferably is in the range from 0.565 to 113 g/l, more preferably from 2.825 to 56.5 g/l, and still more preferably from 5.65 to 28.25 g/l, in terms of the amount of Hb, for example. The amount of the whole blood sample in the reaction system may satisfy both the above-described percent by volume and percent by weight of Hb, or may satisfy either one of them, for example.

The whole blood may be either whole blood collected from a living organism or whole blood treated after being collected, for example. Specifically, the whole blood may be any of hemolyzed whole blood, non-hemolyzed whole blood, anticoagulated whole blood, and whole blood containing coagulated fractions.

In the amplification step, a nucleic acid in the sample may be either a single-stranded nucleic acid or a double-stranded nucleic acid, for example. The nucleic acid in the sample is DNA, for example. The DNA may be DNA contained inherently in a sample such as a biological sample, or an amplification product of DNA amplified by a nucleic acid amplification method, for example. In the latter case, the DNA may be cDNA synthesized from RNA in the sample, for example. Examples of the RNA in the sample include total RNA and mRNA, and the cDNA can be synthesized from the RNA such as described above by RT-PCR (Reverse Transcription PCR), for example.

In the amplification method of the present invention, for example, it is preferable that albumin is also added to the reaction system before starting the amplification reaction in the amplification step. By the addition of albumin, for example, the above-described influence due to the generation of precipitates or turbidity can be reduced still further and also the amplification efficiency can be further improved. Specifically, for example, it is preferable to add albumin before the amplification step. "Before the amplification step" may be before the double-stranded DNA is dissociated into single-stranded DNAs in the amplification step, for example.

The amount of albumin in the reaction system is not particularly limited. The amount is, for example, in the range from 0.01 to 2 wt%, preferably from 0.1 to 1 wt%, and more preferably from 0.2 to 0.8 wt%. The albumin is not particularly limited, and examples thereof include bovine serum albumin (BSA), human serum albumin, rat serum albumin, and horse serum albumin. Only one kind of albumin may be used, or two or more kinds of albumin may be used in combination.

Next, the amplification method of the present invention will be described with reference to an example in which two target regions in a MTHFR gene are amplified at the same time in a single reaction solution by carrying out PCR with DNA in a whole blood sample as the template using, as the MTHFR gene amplification primer set of the present invention, a primer set including primer sets (1) and (2). The two target regions are a target region including a site at which the polymorphism MTHFR*677 occurs and a target region including a site at which the polymorphism MTHFR*1298 occurs. The present invention may be characterized in that the MTHFR gene amplification primer set of the present invention is used, and other configurations and conditions are by no means limited.

First, a PCR reaction solution is prepared. The reaction solution contains the primer set and the whole blood sample, and preferably further contains other components that can be used in the amplification reaction as appropriate.

The amount of each primer in the reaction solution is not particularly limited. The amount of each of the F1 primer and the F2 primer in the reaction solution preferably is 0.1 to 2 µmol/l, more preferably 0.25 to 1.5 µmol/l, and particularly preferably 0.5 to 1 µmol/l. Furthermore, the amount of each of the R1 primer and the R2 primer in the reaction solution preferably is 0.1 to 2 µmol/l, more preferably 0.25 to 1.5 µmol/l, and particularly preferably 0.5 to 1 µmol/l. In primer sets (1) and (2), the ratio of the F primer and the R primer (F : R, molar ratio) is not particularly limited, and is, for example, 1 : 0.25 to 1 : 4, preferably 1 : 0.5 to 1 : 2.

The amount of the whole blood sample in the reaction solution is not particularly limited, and preferably is in the above-described range, for example. The whole blood sample may be added to the reaction solution without being treated, or may be added to the reaction solution after being diluted with a solvent beforehand, for example. Examples of the solvent include water and buffer solutions. When the whole blood sample is diluted beforehand, the dilution ratio is not particularly limited, and can be set so that the final amount of the whole blood in the reaction solution falls within the above-described range, for example. Specifically, the dilution ratio is, for example, 100- to 2000-fold, preferably 200- to 1000-fold.

The above-described other components are not particularly limited, and they may be conventionally known components. The amount of each component is not particularly limited. Examples of the components include solvents. Also, the components may be various components used in PCR, and specific examples thereof include: polymerases such as DNA polymerase; and nucleoside triphosphate. Other examples of the components include albumin, as described above. The order of adding the respective components to the reaction solution is by no means limited.

The polymerase is not particularly limited, and conventionally known polymerases derived from heat-resistant bacteria can be used, for example. As specific examples of such polymerases, *Thermus aquaticus*-derived DNA polymerases (U.S. Patent Nos. 4,889,818 and 5,079,352) (Taqpolymerase™), *Thermus thermophilus-*derived DNA polymerase (WO 91/09950) (rTth DNA polymerase), *Pyrococcus* furiosus-derived DNA polymerase (WO 92/9689) (Pfu DNA polymerase: Stratagenes), *Thermococcus litoralis-derived* polymerase (EP-A 455 430 (Vent™): New England Biolabs), and the like, for example, are commercially available. Among them, *Thermus aquaticus*-derived heat-resistant polymerases are preferable.

The amount of the DNA polymerase in the reaction solution is not particularly limited, and is, for example, 1 to 100 U/ml, preferably 5 to 50 U/ml, and more preferably 20 to 30 U/ml. With regard to the unit of activity (U) of DNA polymerases, 1 U generally is defined as the activity for incorporating 10 nmol of total nucleotides into an acid-insoluble precipitate at 74°C in 30 minutes in a reaction solution for activity measurement using activated salmon sperm DNA as a template primer. The composition of the reaction solution for activity measurement is as follows, for example: 25 mmol/l TAPS buffer (pH 9.3, 25°C), 50 mmol/l KCl, 2 mmol/l MgCl₂, 1 mmol/l mercaptoethanol, 200 µmol/l dATP, 200 µmol/l dGTP, 200 µmol/l dTTP, 100 µmol/l [α-³²P] dCTP, and 0.25 mg/mL activated salmon sperm DNA.

The nucleoside triphosphate generally is dNTP (dATP, dCTP, dGTP, and dTTP or dUTP). The amount of dNTP in the reaction solution is not particularly limited, and is, for example, 0.01 to 1 mmol/l, preferably 0.05 to 0.5 mmol/l, and more preferably 0.1 to 0.3 mmol/l.

Examples of the solvent include buffer solutions and water. Examples of the buffer solution include Tris-HCl, Tricine, MES, MOPS, HEPES, and CAPS, and it is possible to use commercially available buffer solutions for PCR and buffer solutions included in commercially available PCR kits.

As the above-described other components, the reaction solution further may contain heparin, betaine, KCl, MgCl₂, MgSO₄, glycerol, and the like, and the amounts of these components may be set within ranges where they do not interfere with the PCR reaction.

The total volume of the reaction solution is not particularly limited, and can be set as appropriate depending on the device to be used, such as a thermal cycler, and the like, for example. Generally, the total volume is 1 to 500 µl, preferably 10 to 100 µl.

Next, PCR is conducted. The cycle conditions of the PCR are not particularly limited. As a specific example, conditions shown in Table 3 below can be employed, respectively, for (1) dissociation of a double-stranded DNA into single-stranded DNAs; (2) annealing of the primer to the single-stranded DNA; and (3) elongation of the primer, for example. The number of cycles in the PCR is not particularly limited. It preferably is 30 cycles or more with the three steps described in the following items (1) to (3) as one cycle, for example. The upper limit of the number of cycles is not particularly limited, and is, for example, 100 cycles or less, preferably 70 cycles or less, and more preferably 50 cycles or less. The temperature change in each of the steps can be controlled automatically using a thermal cycler or the like, for example. By using the MTHFR gene amplification primer set according to the present invention, high amplification efficiency can be achieved, as described above. Thus, although it takes about 3 hours to perform 50 cycles of treatment in conventional art, it is possible to finish 50 cycles of treatment in, for example, around 1 hour, preferably within 1 hour according to the present invention.

**[Table 3]**

| | Temperature (°C) and Time (seconds) |
|---|---|
| (1) Dissociation into single-stranded DNAs | e.g., 90°C to 99°C, 1 to 120 seconds |
| | preferably, 92°C to 95°C, 1 to 60 seconds |
| (2) Annealing of primer | e.g., 40°C to 70°C, 1 to 300 seconds |
| | preferably, 50°C to 70°C, 5 to 60 seconds |
| (3) Elongation of primer | e.g., 50°C to 80°C, 1 to 300 seconds |
| | preferably, 50°C to 75°C, 5 to 60 seconds |

In the above-described manner, the two target regions in the MTHFR gene can be amplified in the same reaction system at the same time. When either one of the two target regions is amplified, for example, among primer sets (1) and (2), the one that is directed to the target region may be used as the MTHFR gene amplification primer set according to the present invention.

The amplification method of the present invention further may include the step of: detecting an amplification product of the target region obtained in the amplification step. With this configuration, it is possible to detect whether or not the amplification of the target region occurred and to detect the polymorphism, e.g., MTHFR*677 or MTHFR*1298, in the target region in the MTHFR gene, for example, as will be described below. The detection of whether or not the amplification occurred and the detection of the polymorphism can be achieved by conventionally known methods, for example. Specifically, for example, a probe that can hybridize to the detection target site in the MTHFR gene is also added to the reaction system in the amplification step. Examples of the probe include a labeled probe labeled with a fluorescent substance. Then, after the amplification step, in the detection step, the reaction system is subjected to measurement of the fluorescence intensity of the fluorescent substance of the labeled probe. By this, it is possible to determine whether or not amplification of the target region occurred and the polymorphism at the detection target site. Furthermore, when two target regions are to be amplified, for example, two kinds of probes that can hybridize to the respective detection target sites in the MTHFR gene are also added to the reaction system in the amplification step. Then, in the detection step, the reaction system is subjected to measurement of the fluorescence intensity of the fluorescent substance of each of the labeled probes. By this, it is possible to determine whether or not amplification of the respective target regions occurred and the polymorphisms at the respective detection target sites.

The detection of an amplification product of the target region in the MTHFR gene, and the detection of the polymorphisms, e.g., MTHFR*677 and MTHFR*1298, in the MTHFR gene will be described below as additional embodiments of the amplification method of the present invention.

### <Amplification Product Detection Method>

The amplification product detection method according to the present invention includes a method for detecting an amplification product of a MTHFR gene, characterized in that the primer or primer set of the present invention may be used and that the following step (A) is included:
(A) amplifying a MTHFR gene by the amplification method of the present invention.
Step (A) also can be referred to as an amplification step of amplifying a MTHFR gene in a reaction system using the MTHFR gene amplification primer set according to the present invention with a nucleic acid in a sample as the template, for example (the same applies hereinafter).

The detection method of the present invention preferably further includes the step of detecting an amplification product of the MTHFR gene using a probe, for example. Specifically, for example, the detection method preferably includes the following steps (A), (B), and (C):
(A) amplifying a MTHFR gene by the MTHFR gene amplification method according to the present invention;
(B) while changing the temperature of the reaction system containing an amplification product obtained in step (A) and a probe that can hybridize to the amplification product of the MTHFR gene, measuring a signal value indicating the melting state of a hybrid of the amplification product and the probe; and
(C) detecting the amplification product of the MTHFR gene based on the change in the signal value accompanying the temperature change.

In the amplification product detection method of the present invention, regarding step (A), the above description regarding the method for amplifying the MTHFR gene according to the present invention can be referred to.

In the amplification product detection method according to the present invention, the MTHFR gene may be amplified in the presence of the probe, for example. That is, the amplification product detection method according to the present invention may be configured so that, for example, in step (A), a MTHFR gene is amplified in the reaction system containing the probe, and in step (B), the signal values are measured while changing the temperature of the reaction system in step (A), for example.

Regarding the amplification product detection method of the present invention, the description regarding the polymorphism detection method according to the present invention to be described below can be referred to. Specifically, for example, regarding steps (A) and (B) in the amplification product detection method of the present invention, the description regarding steps (A) and (B) in the polymorphism detection method of the present invention to be described below can be referred to. Furthermore, for example, regarding step (C) in the amplification product detection method of the present invention, the description regarding step (D) in the polymorphism detection method of the present invention to be described below can be referred to. Specifically, step (C) also will be described in the following description regarding step (D).

### <Polymorphism Detection Method>

As described above, the polymorphism detection method of the present invention includes a method for detecting a polymorphism in a MTHFR gene, including the following step (A):
(A) amplifying a MTHFR gene by the amplification method of the present invention.

Preferably, the polymorphism detection method of the present invention further includes the step of detecting a polymorphism at a detection target site in the MTHFR gene using a probe, for example. Specifically, for example, the polymorphism detection method preferably includes the following steps (A), (B), and (D):
(A) amplifying a MTHFR gene by the polymorphism detection method according to the present invention;
(B) while changing a temperature of the reaction system containing an amplification product obtained in step (A) and a probe that can hybridize to the detection target site, measuring a signal value indicating the melting state of the hybrid of the amplification product and the probe;
(D) detecting a polymorphism at the detection target site based on the change in the signal value accompanying the temperature change.

By amplifying the MTHFR gene using the primer or primer set of the present invention, it is possible to amplify a target region including the detection target site in the MTHFR gene as described above. This allows the polymorphism at the detection target site in the target region to be analyzed with high sensitivity, for example.

Amplification step (A) can be carried out in the same manner as the above-described amplification method of the present invention. In the amplification product detection method of the present invention, regarding step (A), the above description regarding the MTHFR gene amplification method according to the present invention can be referred to.

In measurement step (B), the probe is not particularly limited. Examples of the probe include a probe that hybridizes to a detection target site of MTHFR*677 or a probe that hybridizes to a detection target site of MTHFR*1298. Hereinafter, the former also is referred to as a "MTHFR*677 probe", and the latter also is referred to as a "MTHFR*1298 probe". These probes preferably are each complementary to a detection target sequence including the detection target site in the MTHFR gene. In the present invention, for example, either one of the MTHFR*677 probe and the MTHFR*1298 probe may be used, or both of them may be used in combination. The probe to be used in the present invention can be determined as appropriate depending on, for example, the kind of target region to be amplified by the MTHFR gene amplification primer or primer set according to the present invention. By using the two kinds of probes in combination, polymorphisms at the two detection target sites, i.e., MTHFR*677 and MTHFR*1298, can be analyzed using the same reaction system, for example.

The probe may be a probe that can hybridize to the sense strand of the MTHFR gene or a probe that can hybridize to the antisense strand of the MTHFR gene, for example. The method for designing the probe is not particularly limited, and a conventionally known method can be employed. For example, a detection target sequence including the detection target site may be set to a sequence in the sense strand of the MTHFR gene, and the probe may be designed based on this sequence. Alternatively, the detection target sequence may be set to a sequence in the antisense strand of the MTHFR gene, and the probe may be designed based on this sequence. The base at the detection target site in the detection target sequence can be determined as appropriate depending on the kind of each polymorphism at the detection target site.

In the case of MTHFR*677, it is known that the 8747th base in SEQ ID NO: 1 is "c" or "t". Thus, for example, the probe may be a probe complementary to a detection target sequence in a sense strand in which the 8747th base is cytosine (c), or a probe complementary to a detection target sequence in a sense strand in which the 8747th base is thymine (t). Such probes each can be referred to as a sense strand detection probe that hybridizes to the sense strand. Examples of the probe also include probes complementary to a sequence in the antisense strand complementary to these sense strands. Such probes each can be referred to as an antisense strand detection probe that hybridizes to the antisense strand.

In the case of MTHFR*1298, it is known that the 10649th base in SEQ ID NO: 1 is "a" or "c". Thus, for example, the probe may be a probe complementary to a detection target sequence in a sense strand in which the 10649th base is adenine (a), or a probe complementary to a detection target sequence in a sense strand in which the 10649th base is cytosine (c). Such probes each can be referred to as a sense strand detection probe that hybridizes to the sense strand. Examples of the probe also include probes complementary to a sequence in the antisense strand complementary to these sense strands. Such probes each can be referred to as an antisense strand detection probe that hybridizes to the antisense strand.

As described above, also by determining the base at a detection target site where the polymorphism occurs to be any of the above-described bases and designing the probe as appropriate, it is possible to determine the polymorphism at each detection target site of the MTHFR gene in a manner to be described below.

The length of the probe is not particularly limited, and is, for example, 5- to 50-mer, preferably 10- to 30-mer.

Specific examples of the probe are, for example, a probe including any one of the following oligonucleotides (P1), (P1'), (P2), and (P2'):
(P1) a 17- to 50-mer oligonucleotide having a base sequence complementary to a base sequence including the 8744th to 8760th bases in SEQ ID NO: 1 and
having a base complementary to the 8744th base in its 3' end region (P1') an oligonucleotide having a base sequence complementary to oligonucleotide (P1)
(P2) a 14- to 50-mer oligonucleotide having a base sequence complementary to a base sequence including the 10643rd to 10656th bases in SEQ ID NO: 1 and having a base complementary to the 10643rd base in its 3' end region; and
(P2') an oligonucleotide having a base sequence complementary to oligonucleotide (P2).

The probe may be a probe containing any of the above-described oligonucleotides or a probe consisting of any of the above-described oligonucleotides, for example.

These probes are illustrative examples, and the present invention is by no means limited thereto. Hereinafter, a probe containing oligonucleotide (P1) is referred to as a P1 probe, a probe containing oligonucleotide (P1') is referred to as a P1' probe, a probe containing oligonucleotide (P2) is referred to as a P2 probe, and a probe containing oligonucleotide (P2') is referred to as a P2' probe. These probes also are referred to as MTHFR gene detection probes of the present invention.

The P1 probe and the P1' probe are examples of the MTHFR*677 probe. They are probes for detecting the polymorphism (c/t) of the 8747th base (y) in the base sequence of SEQ ID NO: 1. Oligonucleotide (P1) is complementary to the sense strand of the MTHFR gene, and the polymorphism can be checked by hybridization of oligonucleotide (P1) to the sense strand, for example. Oligonucleotide (P1') is homologous to the sense strand of the MTHFR gene, and the polymorphism can be checked by hybridization of oligonucleotide (P1') to the antisense strand, for example.

As described above, the base length of each of the oligonucleotides (P1) and (P1') is, for example, 17- to 50-mer, preferably 17- to 40-mer, more preferably 17- to 30-mer, and still more preferably 17- to 21-mer.

Oligonucleotide (P1) includes a base (r) to be paired with the 8747th base (y) in the base sequence of SEQ ID NO: 1. The base (r) is guanine (g) or adenine (a). When "r is g" in the P1 probe, oligonucleotide (P1) shows a perfect match with a detection target sequence in the sense strand in which the detection target site (y) is a wild-type base (c). When "r is a", oligonucleotide (P1) shows a perfect match with the detection target sequence in the sense strand in which the detection target site (y) is a mutant base (t). Thus, depending on whether or not the amplification product obtained by the amplification method shows a perfect match with oligonucleotide (P1), it is possible to determine whether the polymorphism at the detection target site is wild-type or mutant type.

Oligonucleotide (P1) has a base complementary to the 8744th base in its 3' end region. The base preferably is any one of the 1st to 4th bases from the 3' end, more preferably any one of the 1st to 3rd bases from the 3' end, and particularly preferably the 1st base (i.e., the 3' end) or the second base from the 3' end. Oligonucleotide (P1) may be an oligonucleotide having the base sequence of any one of SEQ ID NOs: 44 to 48 shown in Table 4 below. In the base sequence of oligonucleotide (P1), the base (r) may be either guanine (g) or adenine (a), and preferably is adenine (a).

**[Table 4]**

| Probe | Sequence | Tm(°C) | SEQ ID NO |
|---|---|---|---|
| | 5'-gcgtgatgatgaaatcgrctc-3' | 53.6 | 44 |
| | 5'-cgtgatgatgaaatcgrctc-3' | 50 6 | 45 |
| P1 probe for MTHFR*677 | 5'-gtgatgatgaaatcgrctc-3' | 47 5 | 46 |
| | 5'-tgatgatgaaatcgrctc-3' | 45.5 | 47 |
| | 5'-gatgatgaaatcgrctc-3' | 43.3 | 48 |
| | 5'-tcaaagacacttkcttcac-3' | 49.8 | 49 |
| | 5'-caaagacacttkcttcac-3' | 48.2 | 50 |
| P2 probe for MTHFR*1298 | 5'-aaagacacttkcttcac-3' | 46 1 | 51 |
| | 5'-aagacacttkcttcac-3' | 44 9 | 52 |
| | 5'-agacacttkcttcac-3' | 43.5 | 53 |
| | 5'-gacacttkcttcac-3' | 41.2 | 54 |

Oligonucleotide (P1') is complementary to oligonucleotide (P1), as described above. In other words, oligonucleotide (P1') is a 17- to 50-mer oligonucleotide having a base sequence homologous to a base sequence including 8744th to 8760th bases in SEQ ID NO: 1 and having the 8744th base in its 5' end region.

When oligonucleotide (P1') is an oligonucleotide having a base sequence complementary to oligonucleotide (P1), it includes the 8747th base (y) in the base sequence of SEQ ID NO: 1. y is cytosine (c) or thymine (t). When "y is c" in the P1' probe, oligonucleotide (P1') shows a perfect match with a detection target sequence in the antisense strand in which the detection target site (r) is a wild-type base (g). When "y is t" in the P1' probe, oligonucleotide (P1') shows a perfect match with a detection target sequence in the antisense strand in which the detection target site (r) is a mutant base (a). Thus, depending on whether the amplification product obtained by the amplification method shows a perfect match with oligonucleotide (P1'), it is possible to determine whether the polymorphism at the detection target site is wild-type or mutant type.

The P2 probe and the P2' probe are examples of the MTHFR*1298 probe. They are probes for detecting the polymorphism (a/c) at the 10649th base (m) in the base sequence of SEQ ID NO: 1. Oligonucleotide (P2) is complementary to the sense strand of the MTHFR gene, and the polymorphism can be checked by hybridization of oligonucleotide (P2) to the sense strand, for example. Oligonucleotide (P2') is homologous to the sense strand of the MTHFR gene, and the polymorphism can be checked by hybridization of oligonucleotide (P2') to the antisense strand, for example.

As described above, the base length of each of the oligonucleotides (P2) and (P2') is, for example, 14- to 50-mer, preferably 14- to 40-mer, more preferably 14- to 30-mer, and still more preferably 14- to 19-mer.

Oligonucleotide (P2) includes a base (k) to be paired with the 10649th base (m) in the base sequence of SEQ ID NO: 1. k is thymine (t), uracil (u), or guanine (g). When "r is t or u" in oligonucleotide (P2), oligonucleotide (P2) shows a perfect match with a detection target sequence in the sense strand in which the detection target site (m) is a wild-type base (a). When "r is g" in oligonucleotide (P2), oligonucleotide (P2) shows a perfect match with a detection target sequence in the sense strand in which the detection target site (m) is a mutant base (c). Thus, depending on whether the amplification product obtained by the amplification method shows a perfect match with oligonucleotide (P2), it is possible to determine whether the polymorphism at the detection target site is wild-type or mutant type.

Oligonucleotide (P2) has a base complementary to the 10643rd base in its 3' end region. The base preferably is any one of the 1st to 4th bases from the 3' end, more preferably any one of the 1st to 3rd bases from the 3' end, and particularly preferably the 1st base (i.e., the 3' end) or the second base from the 3' end. Oligonucleotide (P2) may be an oligonucleotide having the base sequence of any one of SEQ ID NOs: 49 to 54 shown in Table 4 below. In the base sequence of oligonucleotide (P2), the base (k) may be any of thymine (t), uracil (u) and guanine (g), and preferably is guanine (g).

As described above, oligonucleotide (P2') is complementary to oligonucleotide (P2). In other words, oligonucleotide (P2') is a 14- to 50-mer oligonucleotide having a base sequence homologous to a base sequence including 10643rd to 10656th bases in SEQ ID NO: 1 and having the 10643rd base in its 5' end region.

When oligonucleotide (P2') is an oligonucleotide having a base sequence complementary to oligonucleotide (P2), it includes the 10649th base (m) in the base sequence of SEQ ID NO: 1. m is adenine (a) or cytosine (c). When "m is a" in oligonucleotide (P2'), oligonucleotide (P2') shows a perfect match with a detection target sequence in the antisense strand in which the detection target site (k) is a wild-type base (t). When "m is c" in oligonucleotide (P2'), oligonucleotide (P2') shows a perfect match with a detection target sequence in the antisense strand in which the detection target site (k) is a mutant base (g). Thus, depending on whether the amplification product obtained by the amplification method shows a perfect match with oligonucleotide (P2'), it is possible to determine whether the polymorphism at the detection target site is wild-type or mutant type.

Among the above-listed P1 probes, probe (P1-1) composed of the oligonucleotide having the base sequence of SEQ ID NO: 46 is preferable as the MTHFR*677 probe. Among the above-listed P2 probes, probe (P2-1) composed of the oligonucleotide having the base sequence of SEQ ID NO: 52 is preferable as the MTHFR*1298 probe. In Table 4, "Tm (°C)" of each P1 probe means the Tm value (°C) of the hybrid of the sequence in which the base (r) is adenine (a) and a sequence perfectly complementary thereto. It was calculated with the MELTCALC software using the above-described parameters. In Table 4, "Tm (°C)" of each P2 probe means the Tm value (°C) of the hybrid of the sequence in which the base (k) is guanine (g) and a sequence perfectly complementary thereto. It was calculated in the same manner as described above.

The P1 probe and the P2 probe each may be, for example, a probe composed of any oligonucleotides of the sequence identification numbers shown in Table 4, or a probe containing any of the above-described oligonucleotides.

The probe preferably is a labeled probe having a labeling substance, for example. For example, it is preferable that the oligonucleotide is labeled (modified) with the labeling substance. In the oligonucleotide, a site to be labeled with the labeling substance is not particularly limited, and it preferably is the 5' end region or the 3' end region, more preferably the 5' end or the 3' end, for example. As will be described below, in the oligonucleotide, a base to be labeled with the labeling substance preferably is cytosine (c) or guanine (g), more preferably cytosine (c), for example. The base may be labeled directly with the labeling substance, or alternatively, it may be labeled indirectly with the labeling substance, for example. In the latter case, for example, by labeling any site in a nucleotide residue containing the base, the base can be labeled indirectly with the labeling substance.

Each of the oligonucleotides (P1) and (P2) preferably has the labeling substance in its 3' end region. Specifically, the labeling substance preferably is located at the position of the 1st to 4th bases from its 3' end. It is more preferable that any base from the 1st to 4th bases from the 3' end, still more preferably any base from the 1st to 3rd bases from the 3' end, and particularly preferably the 2nd base from the 3' end or the base at the 3' end has the labeling substance, for example. In oligonucleotide (P1), it is preferable that, for example, any of the bases complementary to the 8744th to 8747th bases has the labeling substance, and it is more preferable that the base (c) complementary to the 8744th base has the labeling substance. In oligonucleotide (P2), it is preferable that, for example, any of the bases complementary to the 10643rd to 10646th bases has the labeling substance, and it is more preferable that the base (c) complementary to the 10643rd base has the labeling substance.

Each of the oligonucleotides (P1') and (P2') preferably has the labeling substance in its 5' end region. Specifically, the labeling substance preferably is located at the position of the 1st to 4th bases from its 5' end. It is more preferable that any base from the 1st to 4th bases from the 5' end, still more preferably any base from the 1st to 3rd bases from the 5' end, and particularly preferably the 2nd base from the 5' end or the base at the 5' end has the labeling substance, for example. In oligonucleotide (P1'), it is preferable that, for example, any of the 8744th to 8747th bases has the labeling substance, and it is more preferable that the 8744th base (g) has the labeling substance. In oligonucleotide (P2'), it is preferable that, for example, any of the 10643rd to 10646th bases has the labeling substance, and it is more preferable that the 10643rd base (g) has the labeling substance.

The labeling substance is not particularly limited, and preferably is one that gives off a signal depending on whether the labeled probe is present independently or it forms a hybrid, for example. The kind of signal is not particularly limited, and examples of the signal include fluorescence and colouring. The colouring may be colour development or colour change, for example. When the signal is fluorescence, examples of a signal value include fluorescence intensity. When the signal is colouring, examples of a signal value include reflectance, absorbance, and transmittance. The signal may be given off from the labeling substance directly or indirectly, for example.

The labeling substance is not particularly limited, and examples thereof include fluorescent labeling substances such as a fluorophore. Examples of the fluorescent substance include fluorescein, phosphor, rhodamine, and polymethine dye derivatives. Examples of commercially available fluorescent substances include Pacific Blue® (Molecular Probes), BODIPY FL® (Molecular Probes), FluorePrime™ (Amersham Pharmacia), Fluoredite™ (Millipore Corporation), FAM® (ABI), Cy3™ and Cy5™ (Amersham Pharmacia), and TAMRA® (Molecular Probes). The detection conditions for the fluorescent substance are not particularly limited, and can be determined as appropriate depending on the kind of fluorescent substance to be used, for example. Specifically, Pacific Blue can be detected at a detection wavelength from 450 to 480 nm, for example; TAMRA can be detected at a detection wavelength from 585 to 700 nm, for example; and BODIPY FL can be detected at a detection wavelength from 515 to 555 nm, for example. When such a labeled probe is used, for example, by detecting fluorescence as the signal and measuring the fluorescence intensity as the signal value, hybridization and dissociation can be checked easily based on the change in fluorescence intensity.

As described above, according to the present invention, two target regions in a MTHFR gene can be amplified in the same reaction system, for example. Thus, by using the MTHFR*677 probe and the MTHFR*1298 probe in combination in the same reaction system, the polymorphisms MTHFR*677 and MTHFR*1298 in the MTHFR gene can be identified in the same reaction system. In this case, it is preferable that the respective probes have different labeling substances that are detected under different conditions. When different labeling substances are used as described above, it becomes possible to analyze the polymorphisms at the respective detection target sites separately even in the same reaction system by changing the detection conditions, for example.

When two or more kinds of probes are used in combination, it is preferable that the respective probes have different fluorescent dyes, specifically, fluorescent dyes detectable at different wavelengths, as the labeling substances, for example. When the probes are guanine quenching probes, for example, each of the MTHFR*677 probes (P1 probes) and the MTHFR*1298 probes (P2 probes) shown in Table 4 is such that, for example, its 3' end region, preferably cytosine (c) at the 3' end is labeled with the fluorescent dye. Specifically, it is preferable to label the respective probes with different fluorescent dyes such as TAMRA and BODIPY FL as the fluorescent dyes, for example. When the 5' end of the probe is labeled with a fluorescent dye, the probe further may have a phosphate group added to its 3' end, for example. This can prevent the probe itself from being elongated, for example.

Preferably, the labeled probe is, for example, a labeled probe that shows a signal independently and shows no signal when it forms a hybrid, or a labeled probe that shows no signal independently and shows a signal when it forms a hybrid. When the labeling substance is a fluorescent substance, the labeled probe preferably is a probe that is labeled with the fluorescent substance, shows fluorescence independently, and shows reduced (e.g., quenched) fluorescence when it forms a hybrid, for example. Such a phenomenon generally is called fluorescence quenching. Probes utilizing this phenomenon generally are called fluorescence quenching probes. Among these fluorescence quenching probes, a preferred probe is one in which the 3' end or the 5' end of the oligonucleotide is labeled with the fluorescent substance, and the base at the end to be labeled is preferably cytosine (c) or guanine (g). In the case where the base at the end is cytosine (c), the base sequence of the fluorescence quenching probe preferably is designed so that, for example, when the fluorescence quenching probe forms a hybrid with a test nucleic acid, the base to be paired with the labeled cytosine (c) at the end or a base away therefrom by one to three bases in the test nucleic acid is guanine (g). A base away from the base to be paired with cytosine (c) by one base is, for example, a base located next to the base to be paired with cytosine (c). Such a probe generally is called a guanine quenching probe, and is known as a so-called QProbe®. When the guanine quenching probe hybridizes to the test nucleic acid, there occurs a phenomenon that, for example, as the fluorescent substance-labeled cytosine (c) at the end approaches guanine (g) in the test nucleic acid, light emission of the fluorescent substance becomes weak, in other words, the fluorescence intensity is reduced. By using the probe such as described above, hybridization and dissociation can be checked easily based on the change in fluorescence intensity, for example. Similarly, in the case where the above-described base at the end is guanine (g), the base sequence of the fluorescence quenching probe preferably is designed so that, for example, when the fluorescence quenching probe forms a hybrid with a test nucleic acid, the base to be paired with the labeled guanine (g) at the end or a base away therefrom by one to three bases in the test nucleic acid is cytosine (c).

The probe may have a phosphate group added to its 3' end, for example. As described above, the amplification step may be carried out in the presence of the probe, for example. In such a case, when the 3' end of the probe has a phosphate group added thereto, it is possible to sufficiently prevent the probe itself from being elongated in the amplification step. A similar effect is obtained also by adding a labeling substance such as described above to the 3' end of the probe, for example.

In the reaction system in measurement step (B), the amount of the probe is not particularly limited. For example, it is preferable that the amount of one kind of probe is in the range from 10 to 1000 nmol/l, more preferably from 20 to 500 nmol/l.

When the probe is a labeled probe having a fluorescent substance as the labeling substance, an unlabeled probe having the same sequence as the labeled probe may be used in combination in order to adjust the fluorescence intensity to be detected, for example. The unlabeled probe may have a phosphate group added to its 3' end, for example. The molar ratio between the labeled probe and the unlabeled probe preferably is 1 : 10 to 10 : 1, for example. In the reaction system, the molar ratio between the amplification product and the probe preferably is 1 : 10 to 10 : 1, for example. The molar ratio between the amplification product and the probe may be the molar ratio between a double-stranded nucleic acid and the probe or the molar ratio between a single-stranded nucleic acid and the probe, for example.

In measurement step (B), it is only necessary that the probe is contained in the reaction system containing the amplification product obtained in amplification step (A), for example, and the timing of adding the probe is not particularly limited. The reaction system in measurement step (B) may be newly prepared using amplification product obtained in amplification step (A) and the probe, or may be the reaction system of amplification reaction in step (A), for example. In the latter case, the probe may be added to the reaction system of the amplification reaction before or during amplification step (A). Alternatively, the probe may be added to the reaction system of the amplification reaction after amplification step (A). In particular, it is preferable that the probe is added to the reaction system of the amplification reaction before the amplification reaction in amplification step (A), for example. In this case, in amplification step (A), it is preferable that the MTHFR gene is amplified in the presence of the probe. With this configuration, for example, it is possible to eliminate the necessity to expose the reaction system to the external environment during or after the amplification reaction in order to add the probe. Also, it is possible to carry out the amplification reaction and the measurement of signal values successively.

As described above, as the reaction system in measurement step (B), the reaction system in amplification step (A) can be used, for example. In the reaction system in amplification step (A), the amount of the sample is not particularly limited, and is as described above in connection with the amplification method of the present invention. In the case where: the sample is a biological sample; the reaction system in amplification step (A) is used as the reaction system in measurement step (B); and optical signal detection is performed in the presence of the probe, it is preferable to set the amount of the sample in the reaction system in amplification step (A) to 0.1 to 0.5 vol%, for example. In the case where the biological sample is a whole blood sample, for example, the amount of the sample in the reaction system preferably is 0.1 to 0.5 vol%, for example. In an amplification reaction such as PCR, a sample generally is subjected to heat treatment to denature the DNA, i.e., dissociation into single-stranded DNAs. However, due to this heat treatment, sugars, proteins, and the like contained in the sample may be denatured, so that precipitates, turbidity, and the like may be generated owing to the insolubility of these components. Thus, when signals are detected in the above-described manner, the generation of such precipitates and turbidity may influence the measurement accuracy. However, for example, by setting the amount of the sample in the reaction system in amplification step (A) within the above-described range, for example, the influence by the generation of the precipitates or the like due to the denaturation can be prevented sufficiently, so that the deterioration of the measurement accuracy of the optical method can be prevented, although the mechanism thereof is unknown. Moreover, by setting the amount of the sample within the above-described range, for example, interference with the amplification reaction by contaminants in the sample also is inhibited sufficiently, so that deterioration of amplification efficiency can be prevented still more effectively. Therefore, by setting the amount of the sample in the reaction system within the above-described range in addition to using the primer or primer set of the present invention, the necessity to pretreat the sample can be eliminated still more effectively.

Furthermore, in the case where the sample is a whole blood sample, the amount of the whole blood sample in the reaction system can also be represented by, for example, percent by weight of hemoglobin (hereinafter referred to as "Hb"), instead of percent by volume (e.g., 0.1 to 0.5 vol%) as in the above. The amount of the whole blood sample in the reaction system preferably is in the range from 0.565 to 113 g/l, more preferably from 2.825 to 56.5 g/l, and still more preferably from 5.65 to 28.25 g/l, in terms of the amount of Hb, for example. The amount of the whole blood sample in the reaction system may satisfy both the above-described percent by volume and the above-described percent by weight of Hb, or may satisfy either one of them, for example.

The polymorphism detection method of the present invention can be utilized in so-called Tm analysis such as described above. The following is an explanation of a Tm value in Tm analysis. For example, when a solution containing double-stranded DNA is heated, absorbance at 260 nm increases. This is caused by the fact that the hydrogen bonds between the strands composing the double-stranded DNA are broken by the heating, whereby the double-stranded DNA is dissociated into single-stranded DNAs (melting of DNA). Then, when every double-stranded DNA is dissociated into single-stranded DNAs, the absorbance of the solution becomes about 1.5 times as large as the absorbance at the time when the heating was initiated (i.e., the absorbance of the solution containing only the double-stranded DNA), whereby it can be determined that the melting is complete. Based on this phenomenon, the melting temperature (Tm) generally is defined as the temperature at the time when the amount of increase in absorbance reaches 50% of the total amount of increase in absorbance.

In measurement step (B), measurement of a signal indicating the melting state of a hybrid of the amplification product and the probe may be, for example, the measurement of absorbance at 260 nm as described above or the measurement of a signal of the labeling substance. Specifically, it is preferable that a labeled probe labeled with the labeling substance is used as the probe as described above and that a signal of the labeling substance is measured. The labeled probe may be, for example, a labeled probe that shows a signal independently and shows no signal when it forms a hybrid, or a labeled probe that shows no signal independently and shows a signal when it forms a hybrid. The former probe does not show a signal when it forms a hybrid (double-stranded DNA) with the amplification product and shows a signal when the probe is dissociated from the amplification product by heating. On the other hand, the latter probe shows a signal when it forms a hybrid (double-stranded DNA) with the amplification product, and the signal is reduced (or quenched) when the probe is dissociated from the amplification product by heating. Therefore, by detecting a signal from the labeling substance, the detection of the progress of the melting of the hybrid, the determination of the Tm value, and the like can be achieved, as in the case where the absorbance at 260 nm is measured. The signal of the labeling substance may be detected under conditions specific to the signal of the labeling substance, for example. Examples of the detection conditions include an excitation wavelength and a detection wavelength. The labeled probe and the labeling substance are as described above.

In step (D) or step (C), detection of the polymorphism based on the change in signal value can be carried out by a conventional method.
Specifically, for example, by comparing the above-described change in signal value with the same regarding a hybrid of the polymorphism detection probe and a mutant detection target sequence and/or a hybrid of the polymorphism detection probe and a wild-type detection target sequence, it is possible to determine whether the polymorphism is mutant type or wild-type. That is, it can be determined that the polymorphism is mutant type when the change in signal value is the same as that of the hybrid with the mutant detection target sequence, whereas it can be determined that the polymorphism is wild-type when the change in signal value is the same as that of the hybrid with the wild-type detection target sequence. Alternatively, for example, it is possible to determine the polymorphism by determining the Tm value based on the change in signal and then comparing the thus-determined Tm value with a Tm value as an evaluation standard. First, the Tm value is determined based on the change in signal value. Then, the measured Tm value is compared with a Tm_{wt} value previously determined for the wild-type target sequence and/or a Tmₘₜ value previously determined for the mutant target sequence. It can be determined that the polymorphism is wild-type when the measured Tm value is the same as or similar to the Tm_{wt} value as the evaluation standard; the polymorphism is mutant type when the measured Tm value is lower than the Tm_{wt} value; the polymorphism is mutant type when the measured Tm value is the same as or similar to the Tmₘₜ value as the evaluation standard; and the polymorphism is wild-type when the measured Tm value is lower than the Tmₘₜ value.

Next, the polymorphism detection method of the present invention will be described with reference to an illustrative example. The present example is directed to the case where a primer set including primer sets (1) and (2) is used as the MTHFR gene amplification primer set according to the present invention and the following two kinds of probes labeled with fluorescent substances are used as the probes. In the present example, two target regions in a MTHFR gene are amplified at the same time in a single reaction solution by PCR using the above-described primer set, and further, two polymorphisms MTHFR*677 and MTHFR*1298 in the MTHFR gene are detected using the probes. The MTHFR*677 probe shown below has the base sequence of SEQ ID NO: 46. The base (r) is adenine (a), and the 3' end of the probe is labeled with a fluorescent dye TAMRA. The MTHFR*1298 probe shown below has the base sequence of SEQ ID NO: 52. The base (k) is guanine (g), and the 3' end of the probe is labeled with a fluorescent dye BODIPY FL. It is to be noted, however, that the present invention is not limited thereto.

(Probe)
MTHFR*677 probe
   5'-gtgatgatgaaatcgActc-(TAMRA)-3' (SEQ ID NO: 55)
MTHFR*1298 probe
   5'-aagacacttGcttcac-(BODIPY FL)-3' (SEQ ID NO: 56)

First, a PCR reaction solution is prepared, and PCR is performed in the above-described manner, thereby amplifying the two target regions in the MTHFR gene at the same time in the same reaction solution. The reaction solution contains primer set (1), primer set (2), and the sample, for example. Preferably, the reaction solution further contains other component(s) that can be used in the amplification reaction as appropriate. According to the present invention, as described above, even a sample containing contaminants, such as whole blood, can be used as it is without being subjected to any pretreatment, for example.

As described above, the reaction solution further may contain the probes, for example. In this case, for example, the MTHFR gene may be amplified in the presence of the probes.

Next, disassociation of the obtained amplification product (double-stranded DNA) into single-stranded DNAs and hybridization of each of the labeled probes with the single-stranded DNA obtained through the dissociation are carried out. This can be achieved by changing the temperature of the reaction solution in the presence of the labeled probes, for example. In this case, as described above, it is preferable that an amplification reaction is carried out in the reaction solution to which the labeled probes have been added, and thereafter, the temperature of the reaction solution is changed.

The heating temperature in the disassociation step may be, for example, a temperature at which the double-stranded amplification product can be disassociated into single strands. The heating temperature is not particularly limited, and is, for example, 85°C to 95°C. The heating time is not particularly limited, and generally is 1 second to 10 minutes, preferably 1 second to 5 minutes.

The hybridization of the labeled probe with the disassociated single-stranded DNA can be achieved by, for example, lowering the heating temperature in the disassociation step after the completion of the disassociation step. The temperature is, for example, 40°C to 50°C. The time period for conducting the treatment at this temperature is not particularly limited, and is, for example, 1 to 600 seconds.

Then, signal values indicating the melting states of the hybrid of the amplification product and the labeled probe are measured while changing the temperature of the reaction solution. Specifically, for example, the reaction solution is heated, in other words, the hybrid of the single-stranded DNA and the labeled probe is heated, and the change in the signal value accompanying the temperature rise is measured. As described above, in the case where a guanine quenching probe, i.e., a probe in which cytosine (c) at the end is labeled, is used, fluorescence is reduced (or quenched) when the probe hybridizes with the single-stranded DNA, and fluorescence is emitted when the probe is disassociated. Therefore, in this case, for example, the hybrid with reduced (quenched) fluorescence may be heated gradually, and the increase in fluorescence intensity accompanying the temperature rise may be measured.

When measuring the change in fluorescence intensity, the temperature range used in the measurement is not particularly limited. The initiation temperature is, for example, room temperature to 85°C, preferably 25°C to 70°C, and the end temperature is, for example, 40°C to 105°C. The temperature rising rate is not particularly limited, and is, for example, 0.1 to 20 °C/sec, preferably 0.3 to 5 °C/sec.

Next, the Tm value is determined by analyzing the change in the signal value. Specifically, from the obtained fluorescence intensities, the amount of change in fluorescence intensity per unit time (-d amount of change in fluorescence intensity/dt or d amount of change in fluorescence intensity/dt) at each temperature is calculated, and the temperature at which the amount of change is largest can be determined as the Tm value. When the labeled probe is the fluorescence quenching probe, the Tm value can be determined in the following manner, for example: the amount of increase in fluorescence intensity is measured, and the temperature at which the amount of increase in fluorescence intensity per unit time (-d amount of increase in fluorescence intensity/dt) is smallest or the temperature at which the amount of increase in fluorescence intensity per unit time (d amount of increase in fluorescence intensity/dt) is largest can be determined as the Tm value. On the other hand, when a probe that shows no signal independently and shows a signal when it forms a hybrid is used as the labeled probe instead of the fluorescence quenching probe, the amount of decrease in fluorescence intensity may be measured, in contrast to the case stated above.

For example, in the case where a plurality of probes respectively labeled with labeling substances that are detected at different detection wavelengths are used as the labeled probes, the change in the signal value may be analyzed at each detection wavelength.

The Tm value can be calculated using MELTCALC software (http://www.meltcalc.com/), which is known conventionally, or the like, for example. Also, the Tm value can be determined by a nearest neighbour method.

In the present example, in order to detect two polymorphisms, MTHFR*677 and MTHFR*1298, signal values are measured under conditions appropriate for the respective labeling substances of the two kinds of probes. Then, based on the signals measured regarding each probe, the Tm value is determined. TAMRA included in the MTHFR*677 probe can be detected at a detection wavelength from 585 to 700 nm, and BODIPY FL included in the MTHFR*1298 probe can be detected at a detection wavelength from 515 to 555 nm.

Then, from these Tm values, the genotypes at the respective detection target sites are determined. That is, whether each of the 8747th base and the 10649th base in the base sequence of SEQ ID NO: 1 is wild-type or mutant type is determined. In the Tm analysis, for example, a perfectly complementary hybrid (match) exhibits a higher Tm value indicating dissociation than a hybrid with one or more different bases (mismatch). Therefore, it is possible to determine the polymorphism at the detection target site by determining beforehand the Tm value of a hybrid of the probe with a sequence perfectly complementary thereto and the Tm value of a hybrid of the probe with a sequence perfectly complementary thereto excluding one base. Specifically, this can be achieved in the following manner, for example. In the case where, assuming that the base at the detection target site is mutant type, e.g., the 8747th base in SEQ ID NO: 1 is thymine (t), a probe complementary to a detection target sequence including this mutant base is used, the polymorphism of the amplification product can be determined as a mutant homozygote when the Tm value of the formed hybrid is the same as the Tm value of the perfectly complementary hybrid. On the other hand, when the Tm value of the formed hybrid is the same as the Tm value of the hybrid with a single base difference (lower than the Tm value of the perfectly complementary hybrid), it can be determined that the polymorphism of the amplification product is a wild-type homozygote, e.g., a homozygote in which the 8747th base in SEQ ID NO: 1 is cytosine (c). Furthermore, when both the Tm values are detected, it can be determined that the polymorphism of the amplification product is a heterozygote. In the above-described manner, from the two Tm values determined regarding the respective probes, the genotypes of the polymorphisms MTHFR*677 and MTHFR*1298 can be determined.

Since the polymorphism in the MTHFR gene can be detected from the change in signal value, it is also possible to detect whether or not the target region including the polymorphism is amplified in the same manner. Thus, by carrying out the above-described step (C) of the amplification product detection method of the present invention in the same manner as step (D), it is possible to detect the amplification product.

In the present invention, instead of raising the temperature of the reaction system containing the probe, in other words, heating the hybrid, and then measuring the change in signal accompanying the temperature rise as described above, the change in signal at the time of hybrid formation may be measured, for example. That is, the temperature of the reaction system containing the probe may be lowered to cause a hybrid to be formed, and the change in signal accompanying the lowering of the temperature may be measured, for example.

As a specific example, the case where a labeled probe that shows a signal independently and shows no signal when it forms a hybrid (e.g., a guanine quenching probe) is used will be described. In this case, the labeled probe emits fluorescence when a single-stranded DNA and the labeled probe are dissociated, and the fluorescence is reduced (or quenched) when the temperature is lowered to allow the labeled probe to form a hybrid. Therefore, for example, the temperature of the reaction system may be lowered gradually, and the decrease in fluorescence intensity accompanying the lowering of the temperature may be measured. On the other hand, when a labeled probe that shows no signal independently and shows a signal when it forms a hybrid is used, the labeled probe does not emit fluorescence when the single-stranded DNA and the labeled probe are dissociated, and the labeled probe emits fluorescence when the temperature is lowered to allow the labeled probe to form a hybrid. Therefore, for example, the temperature of the reaction system may be lowered gradually, and the increase in fluorescence intensity accompanying the lowering of the temperature may be measured.

In the present invention, when only one polymorphism out of the two kinds of polymorphisms, namely, MTHFR*677 and MTHFR*1298, in the MTHFR gene is analyzed, for example, the MTHFR gene amplification primer set of the present invention including, among primer sets (1) and (2), the one that is directed to the target region may be used, and one kind of probe that hybridizes to the detection target site may be used.

### <MTHFR Gene Amplification Reagent>

As described above, the MTHFR gene amplification reagent according to the present invention includes a reagent for amplifying a MTHFR gene, containing the MTHFR gene amplification primer or primer set according to the present invention. The MTHFR gene amplification reagent of the present invention may be characterized in that it contains the primer set of the present invention, and other compositions and the like are by no means limited.

The MTHFR gene amplification reagent of the present invention preferably contains either one of primer set (1) and primer set (2), more preferably both of them, for example.

Preferably, the MTHFR gene amplification reagent of the present invention further contains a probe that can hybridize to an amplification product of a MTHFR gene in order to detect the amplification product obtained by the amplification method using the primer set of the present invention, for example. As described above, according to the primer set of the present invention, depending on the kind of primer sets (1) and (2) included therein, an amplification product of one or two target regions in the MTHFR gene can be obtained, for example. Thus, for example, whether or not the amplification occurred, the polymorphism at the detection target site, etc. can be detected by the probe in the above-described manner. The probe is as described above. The probe preferably includes at least one of the P1 probe and the P2 probe, more preferably both of them, for example.

The MTHFR gene amplification reagent of the present invention preferably is used when amplifying a MTHFR gene in a biological sample such as whole blood, for example. In particular, when the MTHFR gene amplification reagent of the present invention is used in combination with the probe, it is preferable to set the amount of the whole blood sample in the reaction system of an amplification reaction to 0.1 to 0.5 vol%, for example.

The MTHFR gene amplification reagent of the present invention further may contain components necessary for a nucleic acid amplification reaction, for example. Specific examples of such components include: polymerases such as DNA polymerases; nucleoside triphosphates; buffer solutions; and various kinds of catalysts, such as those described above. In the polymorphism detection reagent of the present invention, the respective components may be contained in the same container or separate containers, for example.

The form of the MTHFR gene amplification reagent of the present invention is not particularly limited. For example, it may be in the form of a liquid reagent containing the MTHFR gene amplification primer set according to the present invention, or in the form of a dry reagent containing the same to be suspended in a solvent before use. The content of the MTHFR gene amplification primer set also is not particularly limited.

The amplification reagent of the present invention also can be referred to as a kit for use in the amplification of a MTHFR gene, for example. In the amplification kit of the present invention, the respective components may be contained in the same container or separate containers, for example. The amplification kit of the present invention further may include instructions for use.

### <Amplification Product Detection Reagent and Polymorphism Detection Reagent>

The amplification product detection reagent of the present invention includes a reagent for detecting an amplification product of a MTHFR gene, containing the MTHFR gene amplification primer set according to the present invention. Furthermore, the polymorphism detection reagent of the present invention is a reagent for detecting a polymorphism in a MTHFR gene, containing the MTHFR gene amplification primer set according to the present invention. The reagents of the present invention are characterized in that they contain the MTHFR gene amplification primer set according to the present invention, and other configurations and conditions are by no means limited. Unless otherwise stated, the amplification product detection reagent and polymorphism detection reagent according to the present invention are the same as the MTHFR gene amplification reagent.

The polymorphism detection probe of the present invention is as described above, and is characterized in that it includes any one of the oligonucleotides (P1) and (P2). The polymorphism detection method of the present invention may be a method for detecting a polymorphism in a MTHFR gene, characterized in that it includes the step of detecting a polymorphism in a MTHFR gene using the polymorphism detection probe of the present invention. Regarding the probe of the present invention and various methods using the same, the above descriptions can be referred to.

Next, examples of the present invention will be described. It is to be noted, however, that the present invention is by no means limited by the following examples. In the following examples, "%" means "w/v%", unless otherwise stated.

### Examples

### [Example 1]

In the present example, using a whole blood sample that had not been pretreated as a sample, amplification of a MTHFR gene and polymorphism analysis were carried out.

Whole blood samples were collected from three subjects using a heparin lithium blood collection tube. The whole blood samples thus collected were used as Samples 1, 2, and 3, respectively. 10 µl of each of the whole blood samples was added to 70 µl of a diluent 1 having the following composition, and they were mixed together. 10 µl of this mixture was added to 70 µl of a diluent 2 having the following composition. Thus, diluted samples were prepared. 17 µl of each diluted sample was added to a lidded tube. The tube was heated at 95°C for 10 minutes with the lid being open, thereby concentrating the diluted sample to about 4 µl. 46 µl of the reaction reagent shown in Table 5 below was added to the concentrated sample. The resultant mixture was used as a PCR reaction solution. The PCR reaction solution was subjected to PCR using a thermal cycler. The PCR was carried out under the following conditions: the PCR reaction solutions were first treated at 95°C for 60 seconds, then were subjected to 50 cycles of treatment with a treatment at 95°C for 1 second and at 65°C for 15 seconds as one cycle, and further were treated at 95°C for 1 second and 40°C for 60 seconds. Subsequently, Tm analysis was performed by heating the PCR reaction solution from 40°C to 75°C at a temperature rising rate of 1°C/3 seconds, and measuring the change in fluorescence intensity with time during the temperature rise. The measurement wavelength was set to 515 to 555 nm (for detection of the fluorescent dye BODIPY FL) and to 585 to 700 nm (for detection of the fluorescent dye TAMRA).

| (Diluent 1) | |
|---|---|
| Concentration | Component |
| 10 mol/l | Tris-HCl (pH 8) |
| 0.1 mmol/l | EDTA |
| 0.05% | NaN₃ |
| 0.3% | SDS |

| (Diluent 2) | |
|---|---|
| Concentration | Component |
| 10 mol/l | Tris-HCl (pH 8) |
| 0.1 mmol/l | EDTA |
| 0.05 % | NaN₃ |

**[Table 5]**

| (Reaction Reagent) | |
|---|---|
| Component | Blended Amount (µl) |
| distilled water | 28.255 |
| 100 mmol/l MgCl₂ | 0.75 |
| 10% NaN₃ | 0.185 |
| 20% BSA | 0.5 |
| 80% glycerol | 1.56 |
| 10 × Gene Taq Buffer (Mg free) | 5 |
| 2.5 mmol/l dNTP | 4 |
| 5 µmol/l MTHFR*677 probe | 2 |
| 5 µmol/l MTHFR*1298 probe | 2 |
| 100 µmol/l MTHFR*677 F1 primer | 0.5 |
| 100 µmol/l MTHFR*677 R1 primer | 0.25 |
| 100 µmol/l MTHFR*1298 F2 primer | 0.5 |
| 100 µmol/l MTHFR*1298 R2 primer | 0.25 |
| 5 U/µl Gene Taq FP** | 0.25 |
| Total | 46 µl |

| | |
|---|---|
| ** trade name "Gene Taq FP": NIPPON GENE | |

### (Probe)

MTHFR*677 probe
   5'-gtgatgatgaaatcgActc-(TAMRA)-3' (SEQ ID NO: 55) MTHFR*1298 probe
   5'-aagacacttGcttcac-(BODIPY FL)-3' (SEQ ID NO: 56)

### (Primer set)

MTHFR*677 F1 primer
   5'-cagggagctttgaggctgacctg-3' (SEQ ID NO: 7)
MTHFR*677 R1 primer
   5'-gatggggcaagtgatgcccatg-3' (SEQ ID NO: 15)
MTHFR*1298 F2 primer
   5'-gctgaaggactactacctcttctacctgaag-3'(SEQ ID NO: 25)
MTHFR*1298 R2 primer
   5'-gcatcactcactttgtgaccattccgg-3' (SEQ ID NO: 38)

In Tm analysis, when the formed hybrid is a perfect match hybrid, the Tm value serving as an evaluation standard is as follows: the Tm value of a hybrid of the MTHFR*677 probe and a perfect match sequence is 62.5°C; the Tm value of a hybrid of the MTHFR*677 probe and a mismatch sequence is 56°C; the Tm value of a hybrid of the MTHFR*1298 probe and a perfect match sequence is 57°C; and the Tm value of a hybrid of the MTHFR*1298 probe and a mismatch sequence is 47°C.

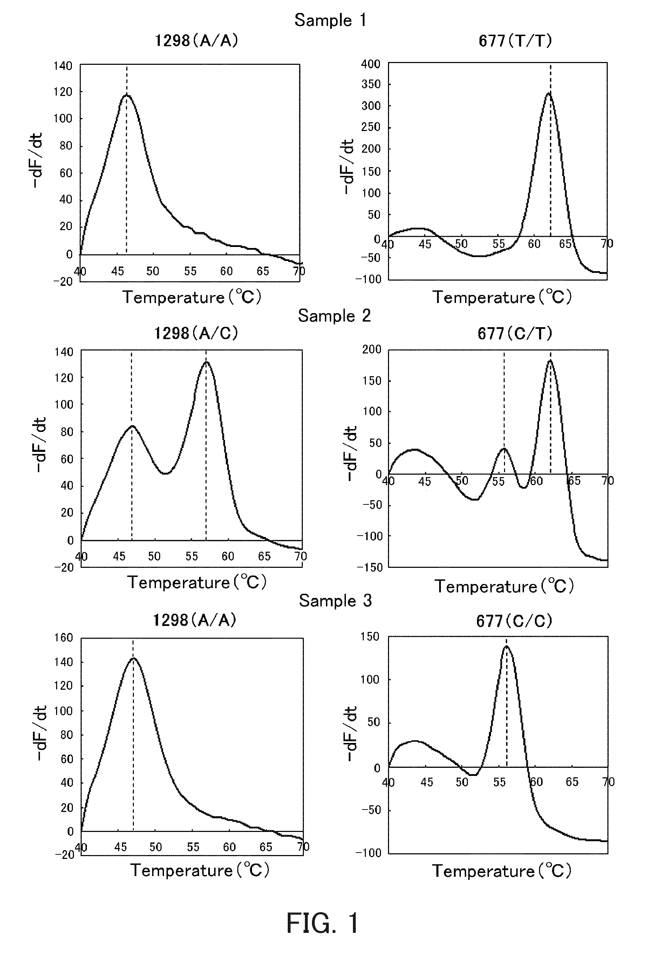

The results are shown in FIG. 1. FIG. 1 shows graphs each showing the result of Tm analysis, indicating the change in fluorescence intensity accompanying the temperature rise. In FIG. 1, the vertical axis indicates the change in fluorescence intensity (hereinafter also referred to as the "amount of change in fluorescence intensity") at each temperature. The unit of the vertical axis is the differential value "-d amount of increase in fluorescence intensity/dt", which is indicated with "-dF/dt". In FIG. 1, the horizontal axis indicates the temperature (°C) at the time of the measurement.

Based on signal peaks shown in FIG. 1, the genotypes of MTHFR*677 and MTHFR*1298 in each sample were determined. As a result, in sample 1, the genotype of MTHFR*677 was homozygote 677 (T/T) with mutant alleles, and the genotype of MTHFR*1298 was homozygote 1298 (A/A) with wild-type alleles. In sample 2, the genotype of MTHFR*677 was heterozygote 677 (C/T) with wild-type and mutant alleles, and the genotype of MTHFR*1298 was heterozygote 1298 (A/C) with wild-type and mutant alleles. In sample 3, the genotype of MTHFR*677 was homozygote 677 (C/C) with wild-type alleles, and the genotype of MTHFR*1298 was homozygote 1298 (A/A) with wild-type alleles.

In order to confirm the results obtained in Example 1, the genotypes of MTHFR*677 and MTHFR*1298 in samples 1, 2, and 3 were determined by a conventional RFLP method. The genotypes determined by the conventional RFLP method regarding the respective samples were the same as those determined in Example 1.

As described above, by using the primer set of the present invention, even when the whole blood sample that had not been pretreated was used, two regions in the MTHFR gene could be amplified at the same time in the same reaction solution, and also, two kinds of polymorphisms could be analyzed using the same reaction solution to allow the genotype to be determined.

### [Example 2]

In the present example, using a purified nucleic acid as a sample, amplification of a MTHFR gene and polymorphism analysis were carried out.

4 µl of purified human genome was used instead of 4 µl of the concentrated reagent in Example 1. Then, PCR and Tm analysis were carried out in the same manner as in Example 1, except that 46 µl of the reaction reagent shown in Table 5 was added without heat-treating the human genome. The purified human genome was prepared from each of the whole blood samples used as samples 1, 2, and 3 in Example 1. Specifically, the whole blood samples were purified using a GFX Genomic Blood DNA Purification Kit (GE Healthcare Bio-Sciences) according to the genomic DNA extraction protocol supplied with the kit. Thus, the purified human genomes were prepared.

In Tm analysis, when the formed hybrid is a perfect match hybrid, the Tm serving as an evaluation standard is the same as described above. That is, the Tm value of a hybrid of the MTHFR*677 probe and a perfect match sequence is 62.5°C; the Tm value of a hybrid of the MTHFR*677 probe and a mismatch sequence is 56°C; the Tm value of a hybrid of the MTHFR*1298 probe and a perfect match sequence is 57°C; and the Tm value of a hybrid of the MTHFR*1298 probe and a mismatch sequence is 47°C.

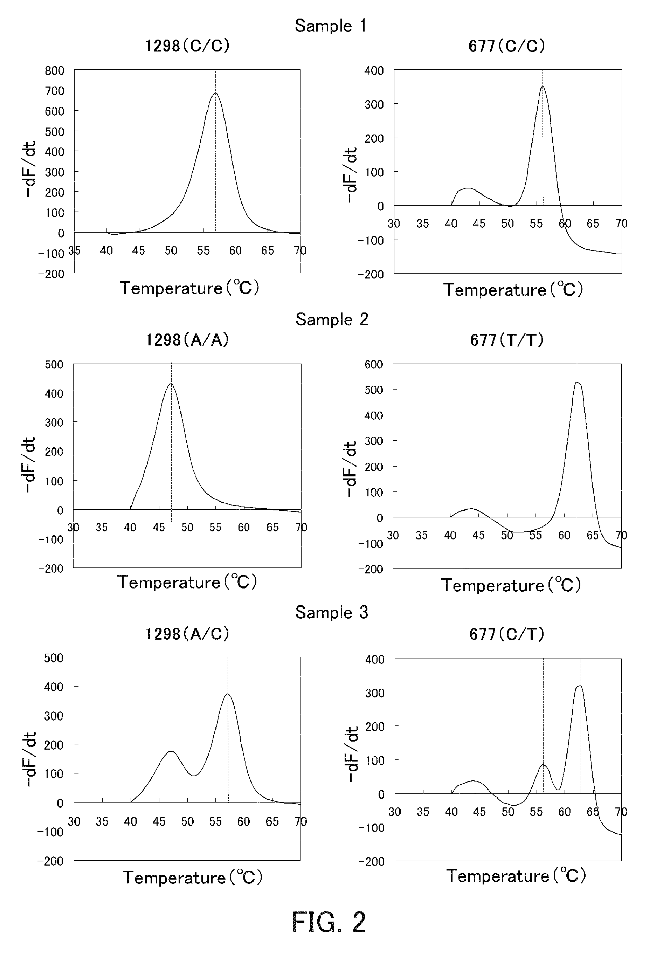

The results are shown in FIG. 2. FIG. 2 shows graphs each showing the result of Tm analysis, indicating the change in fluorescence intensity accompanying the temperature rise. In FIG. 2, the vertical axis indicates the change in fluorescence intensity (hereinafter also referred to as the "amount of change in fluorescence intensity") at each temperature. The unit of the vertical axis is the differential value "-d amount of increase in fluorescence intensity/dt", which is indicated as "-dF/dt". In FIG. 2, the horizontal axis indicates the temperature (°C) at the time of the measurement.

Based on signal peaks shown in FIG. 2, the genotypes of MTHFR*677 and MTHFR*1298 in each sample were determined. As a result, in sample 1, the genotype of MTHFR*677 was homozygote 677 (C/C) with wild-type alleles, and the genotype of MTHFR*1298 was homozygote 1298 (C/C) with mutant alleles. In sample 2, the genotype of MTHFR*677 was homozygote 677 (T/T) with mutant alleles, and the genotype of MTHFR*1298 was homozygote 1298 (A/A) with wild-type alleles. In sample 3, the genotype of MTHFR*677 was heterozygote 677 (C/T) with wild-type and mutant alleles, and the genotype of MTHFR*1298 was heterozygote 1298 (A/C) with wild-type and mutant alleles.

In order to confirm the results obtained in Example 2, the genotypes of MTHFR*677 and MTHFR*1298 in samples 1, 2, and 3 were determined by a conventional RFLP method. The genotypes determined by the conventional RFLP method regarding the respective samples were the same as those determined in Example 2.

As described above, by using the primer set of the present invention, two regions in the MTHFR gene could be amplified in the same reaction solution at the same time, and also, two kinds of polymorphisms could be analyzed using the same reaction solution to allow the genotype to be determined.

### Industrial Applicability

As specifically described above, according to the primer and primer set of the present invention, a target region including a detection target site (e.g., MTHFR*677 or MTHFR*1298) in a MTHFR gene can be amplified specifically. Therefore, according to the primer and primer set of the present invention; a reagent containing the same; and an amplification method and a polymorphism detection method using the primer, primer set, or reagent, it is possible to analyze a polymorphism in a MTHFR gene rapidly and easily. Thus, it can be said that the present invention is very effective in the medical field.

While the present invention has been described with reference to illustrative embodiments and examples, it is to be understood that changes and modifications that may become apparent to those skilled in the art may be made without departing from the scope of the present invention.

This application claims priority from Japanese Patent Application No. 2009-264052 filed on November 19, 2009. The entire disclosure of the Japanese Patent Application is incorporated herein by reference.

## Claims

1. A primer set for amplifying a MTHFR gene, the primer set comprising at least one of:
primer set (1) comprising at least one of a primer composed of the following oligonucleotide (F1) and a primer composed of the following oligonucleotide (R1); and
primer set (2) comprising at least one of a primer composed of the following oligonucleotide (F2) and a primer composed of the following oligonucleotide (R2):
(F1) a 20- to 28-mer oligonucleotide whose 3' end is guanine (g) as the 8715th base in the base sequence of SEQ ID NO: 1;
(R1) an oligonucleotide complementary to an 18- to 26-mer oligonucleotide whose 5' end is cytosine(c) as the 8817th base in the base sequence of SEQ ID NO: 1;
(F2) a 26- to 36-mer oligonucleotide whose 3' end is guanine (g) as the 10590th base in the base sequence of SEQ ID NO: 1; and
(R2) an oligonucleotide complementary to a 22- to 34-mer oligonucleotide whose 5' end is cytosine(c) as the 10695th base in the base sequence of SEQ ID NO: 1.

2. The primer set according to claim 1, wherein the oligonucleotides (F1), (R1), (F2), and (R2) are the following oligonucleotides (F1-1), (R1-1), (F2-1), and (R2-1), respectively:
(F1-1) an oligonucleotide having the base sequence of SEQ ID NO: 7;
(R1-1) an oligonucleotide having the base sequence of SEQ ID NO: 15;
(F2-1) an oligonucleotide having the base sequence of SEQ ID NO: 25; and
(R2-1) an oligonucleotide having the base sequence of SEQ ID NO: 38.

3. A reagent for amplifying a MTHFR gene, the reagent comprising:
the primer set according to claim 1; and
a probe that can hybridize to an amplification product of a MTHFR gene.

4. The reagent according to claim 3, wherein the probe is at least one probe that comprises any one of oligonucleotides (P1), (P1'), (P2), and (P2'):
(P1) a 17- to 50-mer oligonucleotide having a base sequence complementary to a base sequence including the 8744th to 8760th bases in SEQ ID NO: 1 and having a base complementary to the 8744th base in its 3' end region;
(P1') an oligonucleotide having a base sequence complementary to oligonucleotide (P1);
(P2) a 14- to 50-mer oligonucleotide having a base sequence complementary to a base sequence including the 10643rd to 10656th bases in SEQ ID NO: 1 and having a base complementary to the 10643rd base in its 3' end region; and
(P2') an oligonucleotide having a base sequence complementary to oligonucleotide (P2).

5. The reagent according to claim 4, wherein
in oligonucleotide (P1), the base complementary to the 8744th base is any one of the 1st to 4th bases from the 3' end, and
in oligonucleotide (P2), the base complementary to the 10643rd base is any one of the 1st to 4th bases from the 3' end.

6. The reagent according to claim 4, wherein
oligonucleotide (P1) is the following oligonucleotide (P1-1), and oligonucleotide (P2) is the following oligonucleotide (P2-1):
(P1-1) an oligonucleotide having the base sequence of SEQ ID NO: 46; and
(P2-1) an oligonucleotide having the base sequence of SEQ ID NO: 52.

7. The reagent according to claim 3, wherein the probe is a labeled probe having a labeling substance.

8. The reagent according to claim 7, wherein
each of the oligonucleotides (P1) and (P2) has the labeling substance in its 3' end region, and
each of the oligonucleotides (P1') and (P2') has the labeling substance in its 5' end region.

9. The reagent according to claim 8, wherein
each of the oligonucleotides (P1) and (P2) has the labeling substance at the position of the 1st to 4th bases from its 3' end, and
each of the oligonucleotides (P1') and (P2') has the labeling substance at the position of the 1st to 4th bases from its 5' end.

10. The reagent according to claim 8, wherein
in oligonucleotide (P1), the base complementary to the 8744th base has the labeling substance,
in oligonucleotide (P1'), the 8744th base in SEQ ID NO: 1 has the labeling substance,
in oligonucleotide (P2), the base complementary to the 10643rd base has the labeling substance, and
in oligonucleotide (P2'), the 10643rd base in SEQ ID NO: 1 has the labeling substance.

11. The reagent according to claim 4, comprising a probe comprising oligonucleotide (P1) and a probe comprising oligonucleotide (P2).

12. An amplification product detection method for detecting an amplification product of a MTHFR gene, the amplification product detection method comprising the step of:
(A) in a reaction system, amplifying a MTHFR gene using the primer set according to claim 1 with a nucleic acid in a sample as a template.

13. The amplification product detection method according to claim 12, the method comprising the steps of:
(A) in a reaction system, amplifying a MTHFR gene using the primer set according to claim 1 with a nucleic acid in a sample as a template;
(B) while changing the temperature of the reaction system containing an amplification product obtained in step (A) and a probe that can hybridize to the amplification product of the MTHFR gene, measuring a signal value indicating the melting state of the hybrid of the amplification product and the probe; and
(C) detecting the amplification product of the MTHFR gene based on the change in the signal value accompanying the temperature change.

14. The amplification product detection method according to claim 13, wherein,
in step (A), the MTHFR gene is amplified in the reaction system containing the probe, and
in step (B), the temperature of the reaction system in step (A) is changed.

15. The amplification product detection method according to claim 13, wherein the probe comprises any one of the following oligonucleotides (P1), (P1'), (P2), and (P2'):
(P1) a 17- to 50-mer oligonucleotide having a base sequence complementary to a base sequence including the 8744th to 8760th bases in SEQ ID NO: 1 and having a base complementary to the 8744th base in its 3' end region;
(P1') an oligonucleotide having a base sequence complementary to oligonucleotide (P1);
(P2) a 14- to 50-mer oligonucleotide having a base sequence complementary to a base sequence including the 10643rd to 10656th bases in SEQ ID NO: 1 and having a base complementary to the 10643rd base in its 3' end region; and
(P2') an oligonucleotide having a base sequence complementary to oligonucleotide (P2).

16. The amplification product detection method according to claim 15, wherein oligonucleotide (P1) is the following oligonucleotide (P1-1), and oligonucleotide (P2) is the following oligonucleotide (P2-1):
(P1-1) an oligonucleotide having the base sequence of SEQ ID NO: 46; and
(P2-1) an oligonucleotide having the base sequence of SEQ ID NO: 52.

17. The amplification product detection method according to claim 13, wherein the probe is a labeled probe having a labeling substance.

18. The amplification product detection method according to claim 15, wherein the reaction system contains a probe comprising oligonucleotide (P1) and a probe comprising oligonucleotide (P2).

19. A polymorphism detection method comprising the following steps (A), (B), and (D):
(A) in a reaction system, amplifying a MTHFR gene using the primer set according to claim 1 with a nucleic acid in a sample as a template;
(B) while changing the temperature of the reaction system containing the amplification product obtained in step (A) and a probe that can hybridize to a detection target site in the MTHFR gene, measuring a signal value indicating the melting state of the hybrid of the amplification product and the probe;
(D) detecting a polymorphism at the detection target site based on the change in the signal value accompanying the temperature change.

20. The polymorphism detection method according to claim 19, wherein
in step (A), the MTHFR gene is amplified in the reaction system containing the probe, and
in step (B), the temperature of the reaction system in step (A) is changed.

21. The polymorphism detection method according to claim 19, wherein the probe comprises any one of the following oligonucleotides (P1), (P1'), (P2), and (P2'):
(P1) a 17- to 50-mer oligonucleotide having a base sequence complementary to a base sequence including the 8744th to 8760th bases in SEQ ID NO: 1 and having a base complementary to the 8744th base in its 3' end region;
(P1') an oligonucleotide having a base sequence complementary to oligonucleotide (P1);
(P2) a 14- to 50-mer oligonucleotide having a base sequence complementary to a base sequence including the 10643rd to 10656th bases in SEQ ID NO: 1 and having a base complementary to the 10643rd base in its 3' end region; and
(P2') an oligonucleotide having a base sequence complementary to oligonucleotide (P2).

22. The polymorphism detection method according to claim 21, wherein
oligonucleotide (P1) is the following oligonucleotide (P1-1), and oligonucleotide (P2) is the following oligonucleotide (P2-1):
(P1-1) an oligonucleotide having the base sequence of SEQ ID NO: 46; and
(P2-1) an oligonucleotide having the base sequence of SEQ ID NO: 52.

23. The polymorphism detection method according to claim 19, wherein the probe is a labeled probe having a labeling substance.

## Amended claims

### Amended claims under Art. 19.1 PCT

**1.** (Amended) A polymorphism detection probe that can hybridize to a MTHFR gene, the probe comprising at least one of the following labeled oligonucleotides (P1) and (P2) each having a labeling substance:
(P1) a 17- to 50-mer oligonucleotide having a base sequence complementary to a base sequence including the 8744th to 8760th bases in SEQ ID NO: 1 and in which a base complementary to the 8744th base is labeled with a fluorescent dye; and
(P2) a 14- to 50-mer oligonucleotide having a base sequence complementary to a base sequence including the 10643rd to 10656th bases in SEQ ID NO: 1 and in which a base complementary to the 10643rd base is labeled with a fluorescent dye.

**2.** (Amended) The polymorphism detection probe according to claim 1, wherein
oligonucleotide (P1) has the base complementary to the 8744th base at a position of the 1st to 4th bases from its 3' end, and
oligonucleotide (P2) has the base complementary to the 10643rd base at a position of the 1st to 4th bases from the 3' end.

**2.** (Amended) The polymorphism detection probe according to claim 1, wherein
oligonucleotide (P1) has the base complementary to the 8744th base at its 3' end, and
oligonucleotide (P2) has the base complementary to the 10643rd base at its 3' end.

**4.** (Amended) The polymorphism detection probe according to claim 3,
wherein
oligonucleotide (P1) is the following oligonucleotide (P1-1), and oligonucleotide (P2) is the following oligonucleotide (P2-1):
(P1-1) an oligonucleotide having the base sequence of SEQ ID NO: 46; and
(P2-1) an oligonucleotide having the base sequence of SEQ ID NO: 52.

**5.** (Amended) A reagent comprising:
the polymorphism detection probe that can hybridize to a MTHFR gene, according to claim 1.

**6.** (Amended) The reagent according to claim 5, wherein
the reagent comprises a probe comprising oligonucleotide (P1) and a probe comprising oligonucleotide (P2) as the polymorphism detection probes.

**7.** (Amended) The reagent according to claim 5, further comprising:
at least one of the following primer sets (1) and (2) as a primer set for amplifying a MTHFR gene:
Primer set (1):
a primer set comprising at least one of a primer composed of the following oligonucleotide (F1) and a primer composed of the following oligonucleotide (R1):
(F1) a 20- to 28-mer oligonucleotide whose 3' end is guanine (g) as the 8715th base in the base sequence of SEQ ID NO: 1; and
(R1) an oligonucleotide complementary to an 18- to 26-mer oligonucleotide whose 5' end is cytosine(c) as the 8817th base in the base sequence of SEQ ID NO: 1;
Primer set (2):
a primer set comprising at least one of a primer composed of the following oligonucleotide (F2) and a primer composed of the following oligonucleotide (R2):
(F2) a 26- to 36-mer oligonucleotide whose 3' end is guanine (g) as the 10590th base in the base sequence of SEQ ID NO: 1; and
(R2) an oligonucleotide complementary to a 22- to 34-mer oligonucleotide whose 5' end is cytosine(c) as the 10695th base in the base sequence of SEQ ID NO: 1.

**8.** (Amended) The reagent according to claim 7, wherein
the oligonucleotides (F1), (R1), (F2), and (R2) are the following oligonucleotides (F1-1), (R1-1), (F2-1), and (R2-1), respectively:
(F1-1) an oligonucleotide having the base sequence of SEQ ID NO: 7;
(R1-1) an oligonucleotide having the base sequence of SEQ ID NO: 15;
(F2-1) an oligonucleotide having the base sequence of SEQ ID NO: 25; and
(R2-1) an oligonucleotide having the base sequence of SEQ ID NO: 38.

**9.** (Amended) A method for detecting a polymorphism in a MTHFR gene, the method comprising the step of:
detecting a polymorphism in a MTHFR gene using the polymorphism detection probe according to claim 1.

**10.** (Amended) The method according to claim 9, comprising the following steps (A), (B), and (D):
(A) amplifying a MTHFR gene with a nucleic acid in a sample as a template in a reaction system that contains the polymorphism detection probe according to claim 1;
(B) while changing the temperature of the reaction system containing an amplification product obtained in step (A) and the polymorphism detection probe, measuring a signal value indicating the melting state of the hybrid of the amplification product and the polymorphism detection probe; and
(D) detecting a polymorphism at a detection target site based on the change in the signal value accompanying the temperature change.

**11.** (Amended) The method according to claim 10, wherein, in step (A), the MTHFR gene is amplified using at least one of the following primer sets (1) and (2) as a primer set for amplifying a MTHFR gene:
Primer set (1):
a primer set comprising at least one of a primer composed of the following oligonucleotide (F1) and a primer composed of the following oligonucleotide (R1):
(F1) a 20- to 28-mer oligonucleotide whose 3' end is guanine (g) as the 8715th base in the base sequence of SEQ ID NO: 1; and
(R1) an oligonucleotide complementary to an 18- to 26-mer oligonucleotide whose 5' end is cytosine(c) as the 8817th base in the base sequence of SEQ ID NO: 1;
Primer set (2):
a primer set comprising at least one of a primer composed of the following oligonucleotide (F2) and a primer composed of the following oligonucleotide (R2):
(F2) a 26- to 36-mer oligonucleotide whose 3' end is guanine (g) as the 10590th base in the base sequence of SEQ ID NO: 1; and
(R2) an oligonucleotide complementary to a 22- to 34-mer oligonucleotide whose 5' end is cytosine(c) as the 10695th base in the base sequence of SEQ ID NO: 1.

**12.** (Amended) The method according to claim 11, wherein the oligonucleotides (F1), (R1), (F2), and (R2) are the following oligonucleotides (F1-1), (R1-1), (F2-1), and (R2-1), respectively:
(F1-1) an oligonucleotide having the base sequence of SEQ ID NO: 7;
(R1-1) an oligonucleotide having the base sequence of SEQ ID NO: 15;
(F2-1) an oligonucleotide having the base sequence of SEQ ID NO: 25; and
(R2-1) an oligonucleotide having the base sequence of SEQ ID NO: 38.

**13.** (Canceled)

**14.** (Canceled)

**15.** (Canceled)

**16.** (Canceled)

**17.** (Canceled)

**18.** (Canceled)

**19.** (Canceled)

**20.** (Canceled)

**21.** (Canceled)

**22.** (Canceled)

**23.** (Canceled)
